(19)

Europäisches
Patentamt

European
Patent Office

Office européen
des brevets

(11) **EP 4 000 635 A1**

(12) **EUROPEAN PATENT APPLICATION**

(43) Date of publication:
**25.05.2022 Bulletin 2022/21**

(21) Application number: **20207850.7**

(22) Date of filing: **16.11.2020**

(51) International Patent Classification (IPC):
*A61K 47/50* (2017.01)    *C07H 21/02* (2006.01)
*C12Q 1/6806* (2018.01)

(52) Cooperative Patent Classification (CPC):
(C-Sets available)
**A61K 47/549; C12Q 1/6806**          (Cont.)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Hernandez, Frank J.
587 52 Linköping (SE)**

(72) Inventors:
• **HERNANDEZ, Frank J.
587 52 Linköping (SE)**
• **HERNADNEZ, Luiza Iuliana
587 52 Linköping (SE)**
• **BORSA, Baris Ata
58737 Linköping (SE)**

(74) Representative: **Hoffmann Eitle
Hoffmann Eitle S.L.U.
Paseo de la Castellana 140, 3a planta
Edificio LIMA
28046 Madrid (ES)**

(54) **THERAPEUTIC-OLIGONUCLEOTIDES ACTIVATED BY NUCLEASES**

(57)    The present invention refers to a drug delivery system which comprises a polynucleotide sequence susceptible of being cleaved by a specific nuclease derived from a bacterium or a mammalian cell, wherein the polynucleotide sequence, from (upstream to downstream) 5' to 3', comprises i) an oligonucleotide sequence of RNase-resistant modified RNA bases and/or nuclease-resistant DNA bases, acting as a resistant moiety, directly linked to ii) a polynucleotide sequence susceptible of being cleaved by a specific nuclease derived from a bacterium or a mammalian cell which is in turn directly linked to iii) a pharmacological active ingredient, wherein the polynucleotide sequence when cleaved at the cleaving site, releases the pharmacological active ingredient.

EP 4 000 635 A1

(52) Cooperative Patent Classification (CPC): (Cont.)

C-Sets
**C12Q 1/6806**

**Description**

## TECHNICAL FIELD OF THE INVENTION

The present invention pertains to the medical field, in particular to the use of Therapeutic-Oligonucleotides Activated by Nucleases.

## BACKGROUND OF THE INVENTION

Antibiotics are a breakthrough in medical science against pathogenic bacteria.[1] However, their indiscriminate use in the clinic and their abuse in the food industry led to the emergence of antibiotic resistance.[2] This complex issue requires the development of new and innovative antibiotics to overcome this challenge.[3] Targeted therapeutic strategies have been developed mainly for oncological treatments, however, for other human diseases these strategies have been poorly explored.[4] For bacterial infections, several targeted approaches such as fusion-peptides,[5] drug conjugated antibodies,[6] bacteriophages[7] and CRISPRs[8] have been proposed, but none of these have been successfully implemented in the clinical practice.[9] The strategy behind targeted therapeutics is based on the specific recognition of pathogens or sick cells from healthy cells, along with the identification of primary and secondary sites of disease spread. Targeted therapeutics are usually administrated systemically, and the release of the drug is performed with high precision at the disease location.[10] The main benefits of this strategy are the limited or absence of toxicity, and minimal side effects, which improves the safety profile allowing for the treatment of a broader target population. [11,12]

Nucleic acids as therapeutics offers interesting alternatives for the development of targeted drugs. Currently, several nucleoside analogues are being used in the clinical practice and they represent the first line therapeutics for the treatment of many cancers[13] and viral infections.[14] The therapeutic value of the nucleoside analogues have been clearly demonstrated; however better delivery systems need to be developed to formulate them as targeted therapeutics.[15] Previously, the authors of the present invention have developed the TTprobe, an oligonucleotide that consists in a **TT** specific moiety that was flanked by chemically modified 2'-O-Methyl nucleosides (-mC- -mU- or -mG-), acting as the resistant moiety. The **TT** moiety was degraded by micrococcal nuclease (MN), a nuclease produced by *S. aureus* that confers specificity, and the resistant moiety provided protection against non-specific nucleases. The TTprobe was modified at the ends with a fluorophore (FAM) and quencher (RQ) to provide detection capabilities (FAM-mC-mU-mC-mG-**T-T**-mCmGmUmUmC-RQ).[16] Building on this knowledge, we hypothesize that, by incorporating a nucleoside analogue into oligonucleotide substrates that specifically target bacteria (e.g. TTprobe), we could create a novel drug delivery platform for treating bacterial infections in a safer and more efficient manner. Herein, we propose the development of Therapeutic Oligonucleotides Activated by Nucleases (TOUCAN) as a step forward towards the development of targeted therapies. TOUCAN technology combines both, oligonucleotides[16] and nucleoside analogues[17] in a single approach, taking advantage of their best individual performing properties to provide a strategy with great potential for clinical translation. TOUCAN is a first-in-class approach, based on short sequence oligonucleotides programmed as a smart drug delivery system, with the capability to recognize specific bacteria and subsequently release the antibiotic cargo. The TOUCAN concept allows the repurposing of a long list of clinically approved nucleoside analogues[17] to be used as antibiotics.

## BRIEF DESCRIPTION OF THE FIGURES

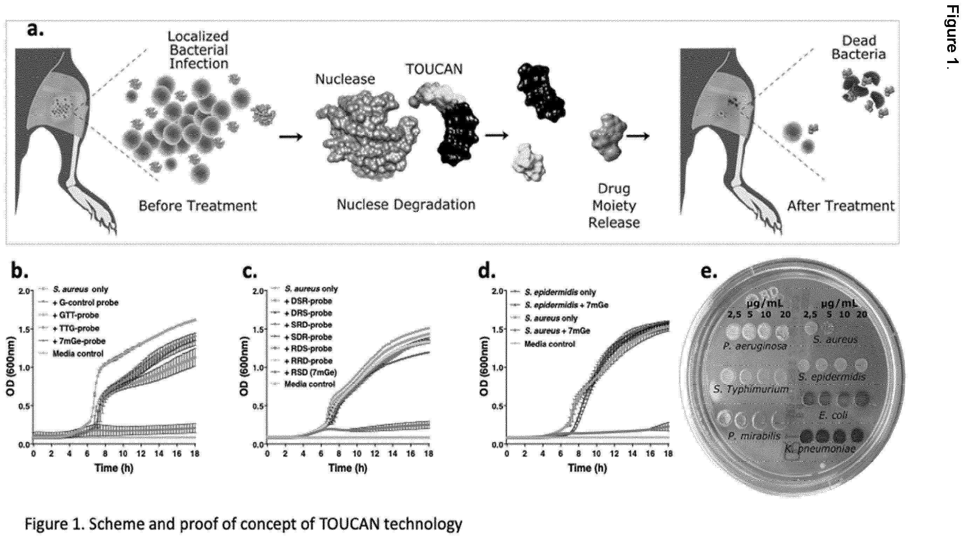

**Figure 1. TOUCAN technology.** a) Schematic representation of TOUCANs mechanism of action in *S. aureus* cultures. b) Growth inhibition curves of TOUCAN with the nucleoside analogue incorporated at different positions. c) Growth curves with all combinations of the three TOUCAN moieties; Resistant (R), Specific (S) and Drug (D). d) Growth curves of TOUCAN for targeted (*S. aureus*) and non-targeted (*S. epidermidis*) bacteria. e) Specificity study of TOUCAN using chromogenic cultures of *S. aureus* and five non-targeted control bacteria. Each data point is the average of at least three replicates and the error bars are one standard deviation.

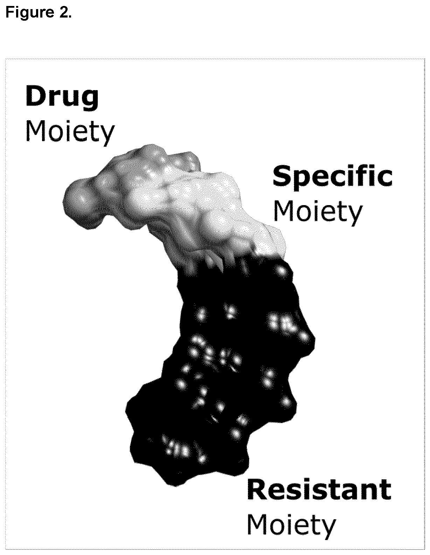

**Figure 2. TOUCAN structure:** TOUCANs consist of three moieties, each having a different function. The resistant moiety (black) confers protection against degradation from non-specific nucleases, while the specific moiety (yellow) allows the targeting of specific bacteria. The drug moiety (green) provides the therapeutic activity.

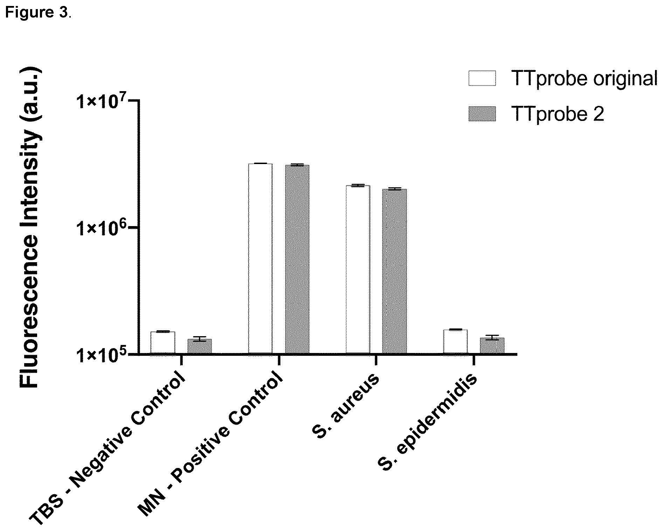

**Figure 3.** Nuclease activity evaluation of TTprobe original and TTprobe2 *(mean; bars ± SD; error bars, n = 3).*

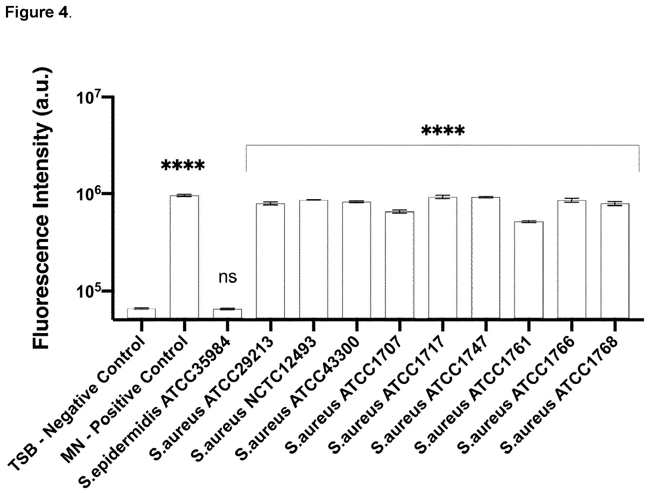

**Figure 4.** Nuclease activity evaluation of all *S. aureus* strains used in this study *(mean; bars ± SD; error bars, n = 3).*

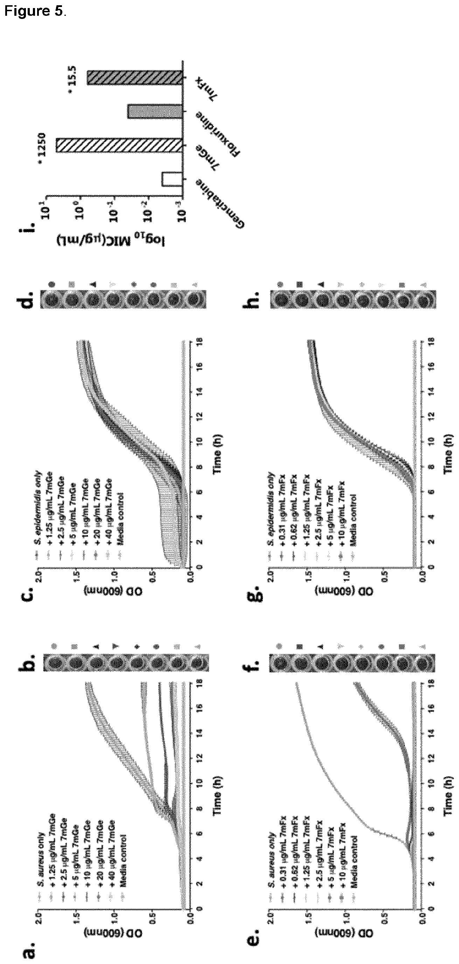

**Figure 5. TOUCANs dose response curves for *S. aureus* susceptible strains.** TOUCAN 7mGe dose response curves and MIC for targeted bacteria (*S. aureus*) along with control bacteria (*S. epidermidis*) evaluated by continuous monitoring of OD at 600nm (**a** and **c**) and by naked eye after 18 hours (**b** and **d**). TOUCAN 7mFx dose response curves and MIC for targeted bacteria (*S. aureus*) along with control bacteria (*S. epidermidis*) evaluated by continuous monitoring of OD at 600nm (**e** and **g**) and by naked eye after 18 hours (**f** and **h**). i) Log MIC values for each treatment: Gemcitabine, 7mGe, Floxuridine and 7mFx. The calculated TERs values are shown on top of each TOUCAN bar and labeled with an asterisk. (Each data point represents the *mean; ± SD; error bars, n = 3*).

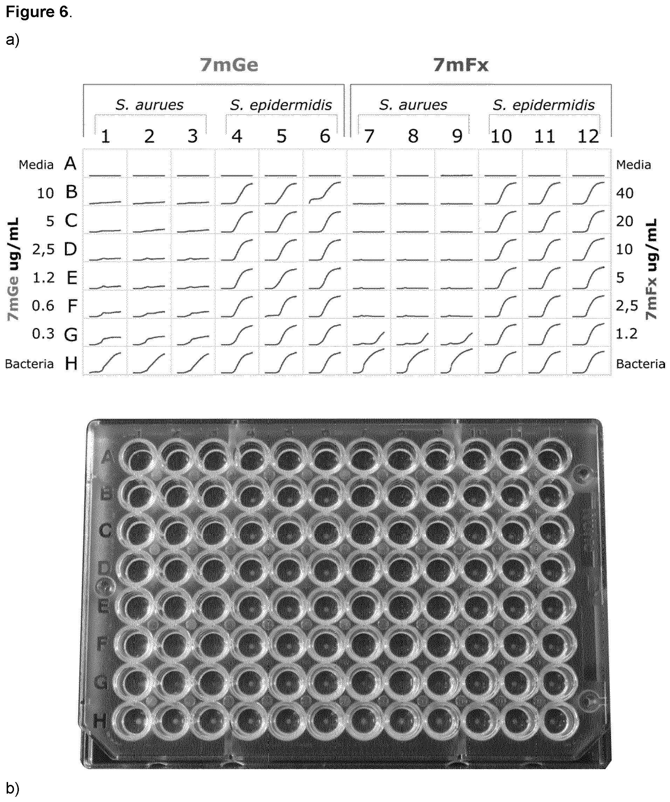

**Figure 6. TOUCANs dose response curves and MICs.** a) Raw data of kinetic dose response analysis of TOUCAN 7mGe and TOUCA 7mFx acquired at 600nm optical density every 10 min for 18 h. b) MICs and visualization by naked eye of the plate after 18 h.

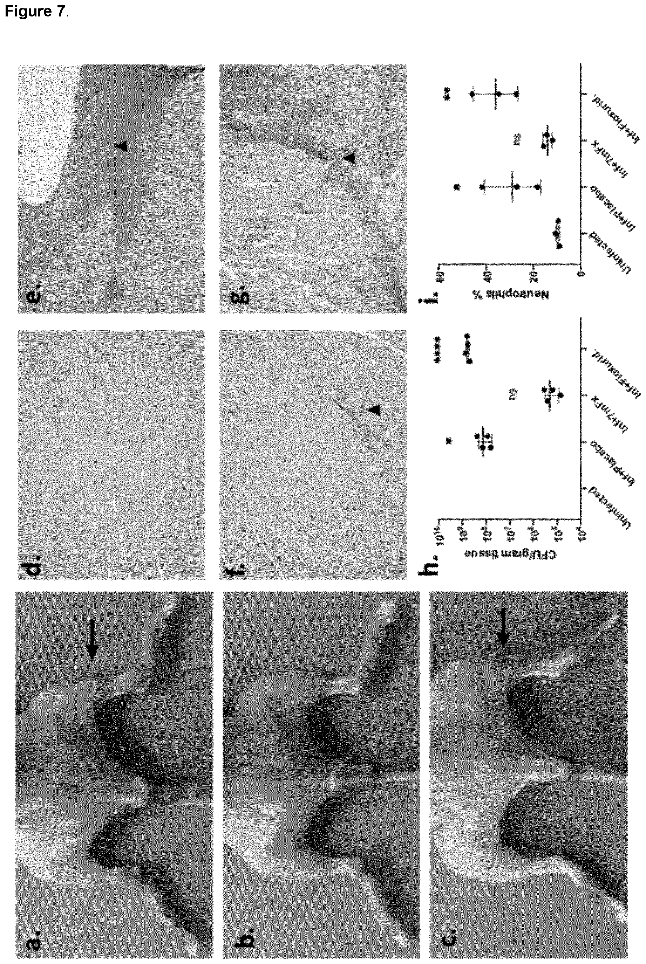

**Figure 7. Therapeutic efficacy of TOUCANs in mice.** Representative gross photographs of mice groups treated with a) PBS, b) TOUCAN 7mFx and c) Floxuridine. Histopathological evaluation of mouse leg tissues of d) uninfected/untreated, e) infected treated with Placebo (PBS), f) infected treated with TOUCAN 7mFx and g) infected treated with Floxuridine. h) Bacteria counts of uninfected and infected legs reported as CFU/g along with i) percentage blood neutrophils of uninfected and infected mice. All photographs are representative of at least 4 mice per group. The values plotted for the bacterial counts and neutrophils are the average of at least 4 and 3 mice, respectively, and the error bars are one standard deviation.

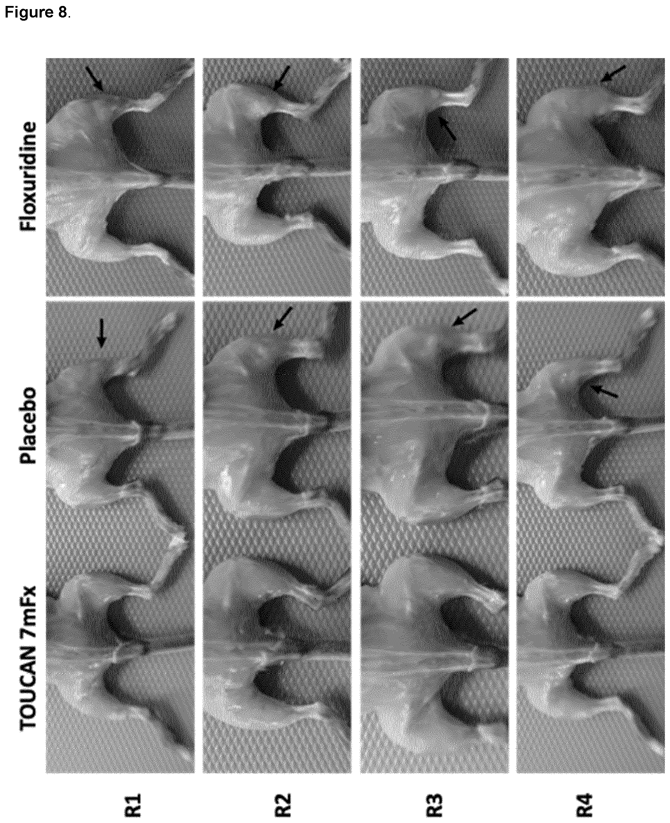

**Figure 8.** Gross replicate photographs of TOUCAN 7mFx, Placebo and Floxuridine groups.

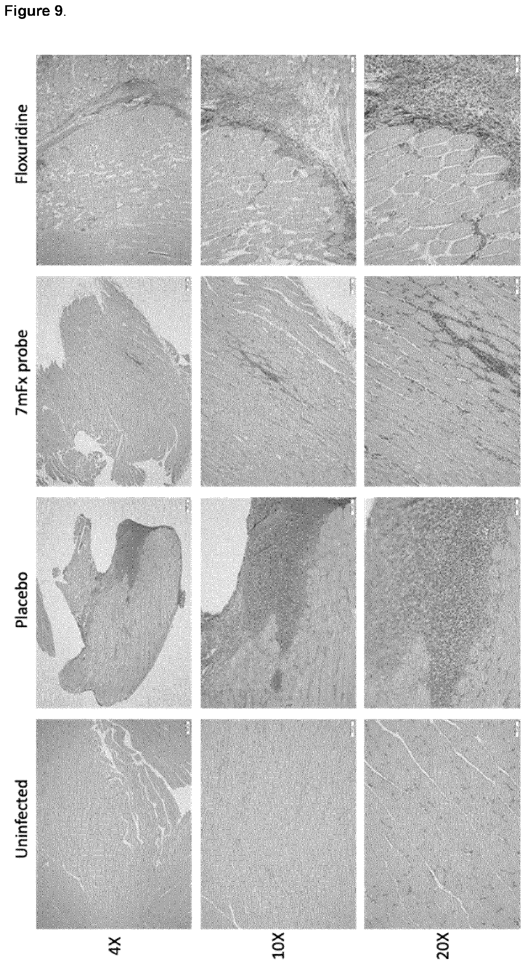

**Figure 9.** Representative histopathological images of uninfected and infected mice using different optical objectives (4x, 10x and 20x).

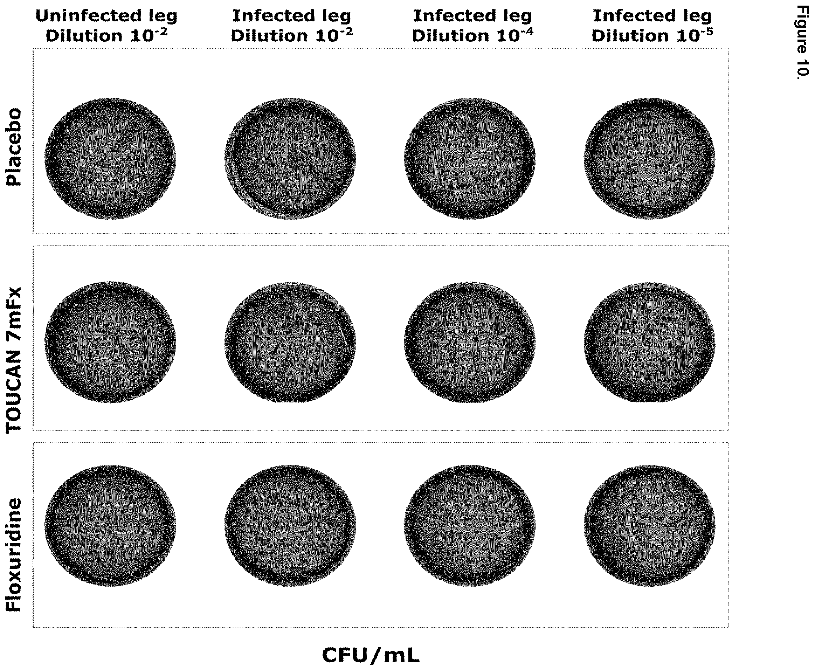

**Figure 10.** Representative photographs of bacterial counts from uninfected legs from all the groups and infected leg treated with Placebo, TOUCAN 7mFx and Floxuridine.

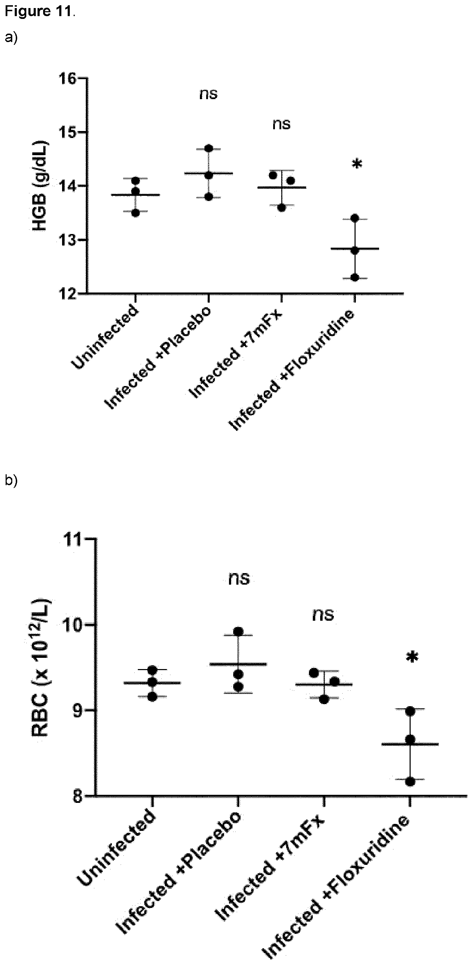

**Figure 11. Evaluation of acute blood toxicity in mice.** a) Red blood cells (RBCs) count, b) percent Hematocrit (HCT) and c) Hemoglobin (HGB) of untreated and treated animal groups. *(mean; bars ± SD; error bars, n = 4).*

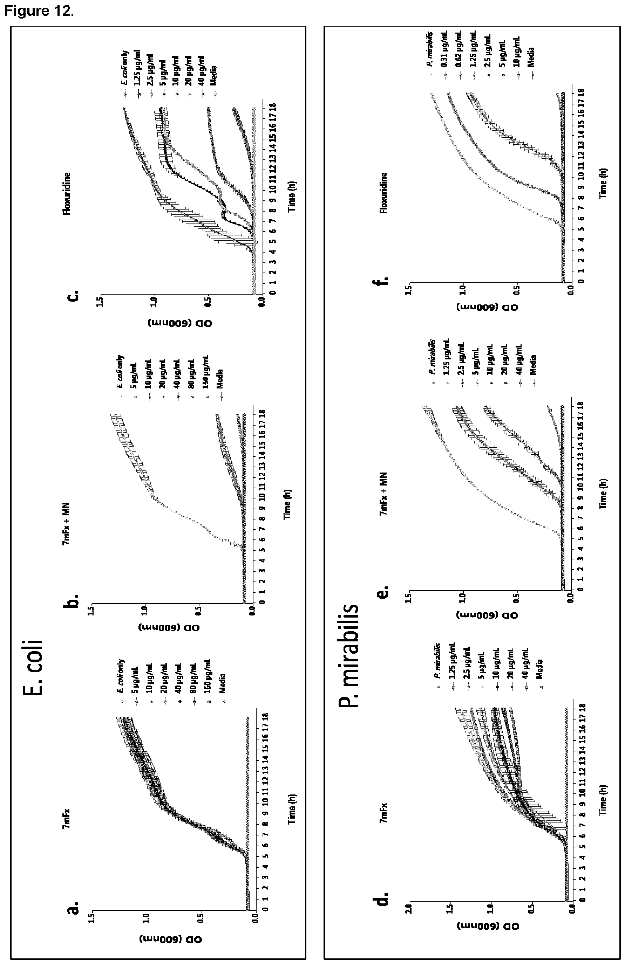

**Figure 12. Therapeutic activity of TOUCANs in gram negative bacteria.** TOUCAN 7mFx dose response curves for *Escherichia coli* (*E. coli)* treated with **a)** 7mFx, **b)** 7mFx spiked with micrococcal nuclease (MN), **c)** Floxuridine alone. TOUCAN 7mFx dose response curves for *Proteus mirabilis* (*P. mirabilis*) treated with **d)** 7mFx, **e)** 7mFx spiked with micrococcal nuclease (MN), **f)** Floxuridine alone. (Each data point represents the *mean; ± SD; error bars, n = 3).*

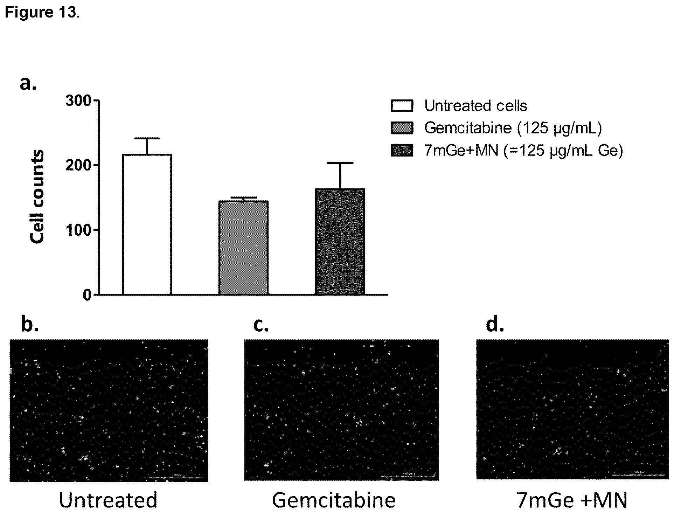

**Figure 13. Evaluation of the therapeutic efficacy of TOUCANs in pancreatic cancer cells.** Viability studies using pancreatic cancer cells. **a)** Pancreatic cancer cell counts after 72h incubation with control (untreated, white bars), Gemcitabine (grey bars) and 7mGe spiked with MN (dark grey). Fluorescence microscopy images were acquired for **b)** Untreated, **c)** Gemcitabine alone and **d)** 7mGe spiked with MN.

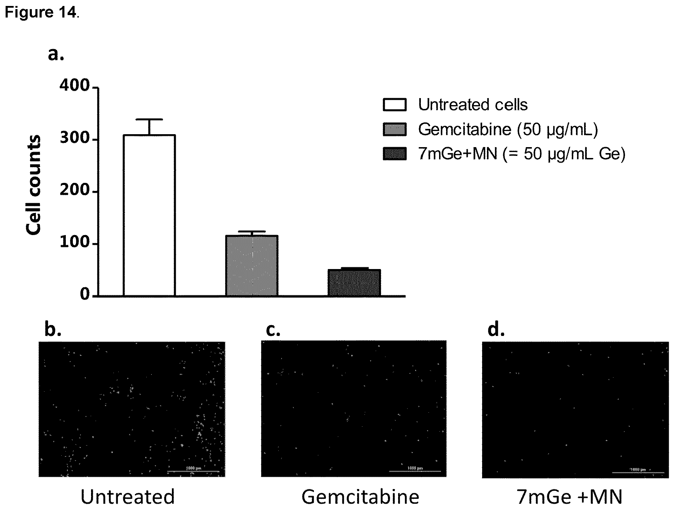

**Figure 14. Evaluation of the therapeutic efficacy of TOUCANs in breast cancer cells.** Viability studies using breast cancer cells. **a)** Breast cancer cell counts after 72h incubation with control (untreated, white bars), Gemcitabine (grey bars) and 7mGe spiked with MN (dark grey). Fluorescence microscopy images were acquired for **b)** Untreated, **c)** Gemcitabine alone and **d)** 7mGe spiked with MN.

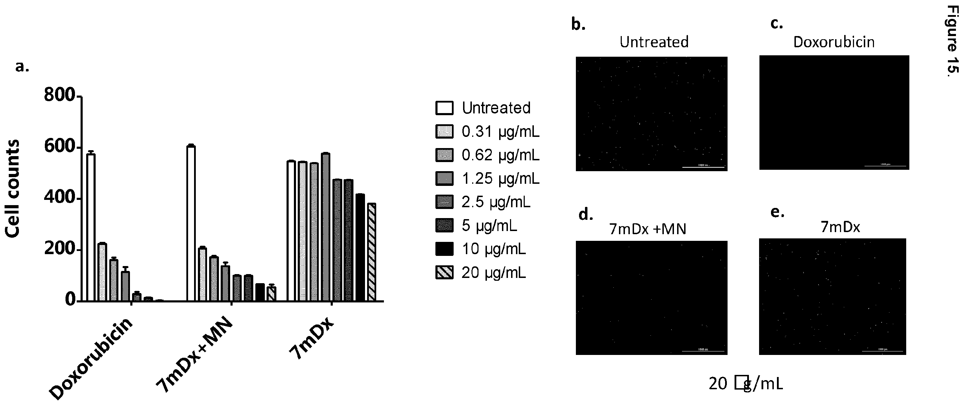

**Figure 15. Therapeutic efficacy of TOUCANs containing Doxorubicin (7mDx). a)** Viability and TOUCAN 7mDx dose response curves for breast cancer after 96h. Fluorescence microscopy images at 20 $\mu$g/mL for **b)** Untreated, **c)** Doxorubicin alone, **d)** 7mDx spiked with MN, and **e)** 7mDx alone.

## DETAILED DESCRIPTION OF THE INVENTION

We herein describe a Therapeutic-Oligonucleotides Activated by Nucleases (TOUCAN) based technology that combines both oligonucleotides and, preferably, nucleoside analogues in a single approach, taking advantage of their best individual performing properties to provide a strategy with great potential for clinical translation.

**Figure 1a** shows the schematic of TOUCAN's mechanism of action in a mouse pyomyositis model. Briefly, once TOUCANs become in the proximity of nucleases (MN), such as those derived from *S. aureus* infections (bacteria in orange), they are degraded, thus releasing the drug moiety. Subsequently, the drug is internalized into bacteria resulting in efficient cell death (bacteria in brown). This pro-drug mechanism can offer an attractive strategy to treat infections. TOUCANs consist of three moieties, each having a different function. The resistant moiety (**Figure 2,** black) confers protection against degradation from non-specific nucleases, while the specific moiety (**Figure 2,** yellow) allows the targeting of specific bacteria. The drug moiety (**Figure 2,** green) is preferably a nucleoside analogue that works as an antibiotic.

In the present invention, we sought to demonstrate that the incorporation of a nucleic acid analogue into the TTprobe,[16] preserves its recognition properties for *S. aureus*. Gemcitabine (Ge) is a nucleoside antimetabolite that is widely used for the treatment of several solid tumors.[18] However, its systemic administration is associated with significant toxicity.[19] Several studies have also demonstrated the antibacterial potential of Gemcitabine by reporting efficient killing of *S. aureus* cultures at low minimal inhibitory concentrations (MICs).[20, 21] We have performed Gemcitabine experiments in our laboratory and we have reproduced the killing curves, obtaining similar MIC values of 0.004 $\mu$g/mL for *S. aureus* (**Table 1**).

Table 1. MICs and bacterial growth inhibition in *S. aureus* strains NCTC 12493 and ATCC 29213 by Gemcitabine, Vidarabine, Floxuridine, Cytarabine, Trifluridine and Idoxuridine nucleoside analogues *(mean ± SD, n = 3).*

| Nucleoside Analogue | Bacteria | | | |
|---|---|---|---|---|
| | S.aureus NCTC 12493 | | S.aureus ATCC 29213 | |
| | MIC (µg/ml) | % Growth inhibition | MIC (µg/ml) | % Growth inhibition |
| Gemcitabine | 0,004 | 93,7±0,2 | 0,004 | 94,2±0,04 |
| Vidarabine | 40 | 85,1±0,8 | >40 | 34,1±0,8 |
| Floxuridine | 0,32 | 87,9±0,4 | 0,08 | 93,1±0,3 |
| Cytarabine | >40 | 39,4±2,5 | >40 | 16,3±6,9 |
| Trifluridine | 10 | 88,5±1,1 | 10 | 89,0±5,1 |
| Idoxuridine | >40 | 46,0±0,6 | >40 | 13,4±3,4 |

Next, to facilitate the incorporation of Ge into the TTprobe, we synthesized Ge in the phosphoramidite format. For this study, we engineered our previously reported TTprobe[16] into a simpler sequence with only two types of nucleosides (UUUUTTUUUU), named TTprobe2, where the U stands for 2'-O-methyl uridine; and T stands for deoxythymidine. To demonstrate that TTprobe2 parallels the efficiency of the original TTprobe, the nuclease activity assay was performed using cultures of *S. aureus* (MN+) and control cultures of *Staphylococcus epidermidis* (*S. epidermidis*), a bacterium that does not produce MN (MN-). Our results confirm the efficient degradation of the TTprobe2 by *S. aureus,* while the probe was rendered stable in the presence of *S. epidermidis* (**Figure 3**). Furthermore, we investigated whether the incorporation of therapeutic nucleoside analogues, such as Ge, into the TTprobe2 can provide therapeutic efficacy against *S. aureus.* Ge nucleoside was incorporated into the TOUCAN structure using two strategies: i) Upstream GeTT (UUUU**GeTT**UUUU), and ii) Downstream TTGe (UUUU**TTGe**UUUU). We also included a Ge-control sequence without the TT motif (UUUU**Ge**UUUU), which was expected to be non-degradable. The efficacy of the specific release was demonstrated in *S. aureus* cultures by measuring the optical density (OD) over 18 h. TOUCANs Ge-control and GeTT have shown low growth inhibition efficiency in *S. aureus* cultures (**Fig 1b**, green and blue lines, respectively), whereas, TTGe inhibited around 20% of bacterial growth, (**Fig 1b**, purple line) indicating that downstream incorporation (TTGe) facilitates the therapeutic activity of Gemcitabine. Based on these results, we designed an additional TOUCAN consisting of the first 7mer of the TTGe, that we named 7mGe (UUUU**TTGe**). The rationale for this design is that TOUCANs will be cleaved at the TT site, releasing the Gemcitabine as a monomer. The 7mGe design was aligned with the therapeutic mechanism of action of Gemcitabine that involves: i) crossing the bacterial membrane, ii) intracellular phosphorylation and iii) inhibition of DNA synthesis.[22-24] We thus expected an increase in the TOUCAN 7mGe's efficacy. TOUCAN 7mGe was synthetized and evaluated under the same conditions as previously described for the other TOUCANs. **Fig 1b** shows the remarkable therapeutic efficacy of TOUCAN 7mGe with the capability of inhibiting 96% of the *S. aureus* growth of (pink line). Next, we sought to interrogate whether the location of each moiety played a role in the functionality of TOUCANs. We hypothesized that the order of these three moieties (resistant moiety; R, specific recognition moiety; S and the drug moiety; D) within the structure of TOUCAN was critical for their therapeutic efficacy. To validate our hypothesis, we synthesized the TOUCAN 7mGe with all possible combinations of the R-S-D moieties, along with the RRD-probe control (without the TT motif). **Figure 1c** reports the therapeutic efficacy of all combinations of the three moieties, clearly indicating that RSD (7mGe) was the only combination with therapeutic efficacy, resulting in more than 99% inhibition of bacterial growth, followed by the SDR-probe with 30% efficacy. **Table 2** provides a detailed sequence description of all combinations analyzed and all the sequences used in this study.

**Table 2.** Oligonucleotides probes and TOUCAN sequences used in this study

| Oligos | Name | Sequence |
|---|---|---|
| 1 | TTprobe original | **FAM**-mC-mU-mC-mG-**T-T**-mC-mG-mU-mC-**TQ2** |
| 2 | TTprobe2 | **FAM**-mU-mU-mU-mU-**T-T**-mU-mU-mU-mU-**TQ2** |
| 3 | GeTT | mU-mU-mU-mU-**Ge-T-T**-mU-mU-mU-mU |
| 4 | TTGe | mU-mU-mU-mU-**T-T**-**Ge**-mU-mU-mU-mU |
| 5 | Ge-Control | mU-mU-mU-mU-**Ge**-mU-mU-mU-mU |
| 6 | RSD (TOUCAN 7mGe) (S.aureus) | mU-mU-mU-mU-**T-T**-**Ge** |
| 7 | DSR probe | **Ge-T-T**-mU-mU-mU-mU |
| 8 | DRS probe | **Ge**-mU-mU-mU-mU-**T-T** |
| 9 | SRD probe | **T-T**-mU-mU-mU-mU-**Ge** |
| 10 | SDR probe | **T-T**-**Ge**-mU-mU-mU-mU |
| 11 | RDS probe | mU-mU-mU-mU-**Ge-T-T** |
| 12 | RRD probe | mU-mU-mU-mU-**Ge** |
| 13 | TOUCAN 7m**Fx** (S. aureus) | mU-mU-mU-mU-**T-T-Fx** |
| 14 | TOUCAN 7m**Dx** (S. aureus) | mU-mU-mU-mU-**T-T-Dx** |
| 15 | TOUCAN 9m**Ge** (cancer) | mU-mU-mU-mU-fAfAfA-**Ge** |
| 16 | TOUCAN 9m**Dx** (cancer) | mU-mU-mU-mU-fAfAfA-**Dx** |
| 17 | TOUCAN 9m**Dx** (cancer) | mU-mU-mU-mU-fCfCfC-**Ge** |
| 18 | TOUCAN 9m**Dx** (cancer) | mU-mU-mU-mU-fCfCfC-**Dx** |
| 19 | **TOUCAN 7mRes (Coronavirus)** | mU-mU-mU-mU-**U**-Rm |
|  |  |  |

**FAM** = 6-Carboxyfluorescein
**TQ2** = Tide Quencher 2
mC = 2'-O-Methyl C
mU = 2'-O-Methyl U
mG = 2'-O-Methyl G
**T** = Deoxythymidine, DNA nucleoside T
**U** = Uridine, RNA nucleoside U
fA = 2'-Fluoro A
fC = 2'-Fluoro C
**Ge** = Gemcitabine
**Fx** = Floxuridine
Dx = Doxorubicin
Rm = Remdesivir

These results confirm that TOUCAN system is a modular approach with three moieties that need to follow a specific order for therapeutic efficacy. Next, we sought to evaluate the safety and specificity of the lead TOUCAN RSD (7mGe), by generating inhibition growth curves with *S. aureus* (target) and *S. epidermidis* (control). **Fig 1d** shows high growth inhibition of 7mGe for *S. aureus* (pink) and minimal associated toxicity to *S. epidermidis* (blue), confirming the safety profile for non-targeted bacteria. Specificity studies were also conducted by visual inspection on chromogenic media using cultures of six other pathogenic bacteria. As indicated in **Figure 1e,** only *S. aureus* was partially inhibited at 5 μg/mL and completely inhibited at concentrations of 10 and 20 μg/mL, while the other bacteria remained viable at all concentrations tested. These results are in agreement with all reported growth inhibition curves in this study and confirm the feasibility and specificity of TOUCANs. One interesting feature of TOUCAN technology is the possibility to change the drug moiety by incorporating other potent nucleoside analogues. Poised by our results, we sought to explore this possibility by testing five nucleoside analogues that have been approved as therapeutic drugs in humans and have similar mode of action as Gemcitabine. With this in mind, we have further evaluated Vidarabine, Floxuridine, Cytarabine, Trifluridine and Idoxuridine against two strains of *S. aureus* (NCTC 12493 and ATCC 29213) that are commonly used as quality control strains for susceptibility testing. MICs values and the percentage of growth inhibition for each nucleoside analogue are reported in **Table 1.** We observed that Gemcitabine and Floxuridine were the only nucleoside analogues able to inhibit the growth of both strains with low MIC values. Thus, we decided to perform further analysis with Gemcitabine (Ge) and Floxuridine (Fx). It has been previously reported that the antibiotic response of different strains within the same species can differ based on several mechanisms. Those mechanisms involve metabolic and growing rates, drug resistance, antimicrobial receptors,[25] and more importantly, in the case of TOUCANs, the functionality of nucleoside transporters[26] and kineses.[27] Gemcitabine and TOUCAN 7mGe were tested against nine common strains of *S. aureus;* from which eight strains are methicillin-resistant *Staphylococcus aureus* (MRSA), and one is methicillin-sensitive *Staphylococcus aureus* (MSSA), along with six non-targeted bacteria used as controls. **Table 3** shows that the effect of Gemcitabine treatment on all *S. aureus* strains tested is consistent with the MIC values from 0.004 to 0.016 μg/mL. In contrast, the TOUCAN 7mGe at lower doses was able to efficiently inhibit the growth of two out of the nine strains tested (85,3% and 90,1%), with MIC values of 5 and 40 μg/mL. The other 7 strains at low doses have reported an inhibition growth in between 44,9% and 86,2%. To improve the therapeutic activity in these 7 strains, a higher dose should be used. These results suggest that TOUCAN 7mGe inhibit the growth of *S. aureus* for all tested strains.

**Table 3.** MICs and bacterial growth inhibition in nine *S. aureus* strains by Gemcitabine and TOUCAN 7mGe *(mean ± SD, n = 3).*

| Bacteria | Nucleoside Analogue (NA) | TOUCAN | TOUCAN | TOUCAN MIC / NA MIC |
|---|---|---|---|---|
|  | Gemcitabine MIC (μg/mL) | 7mGe MIC (μg/mL) | % Growth inhibition | TOUCAN Efficacy Ratio (TER) |
| S.aureus NCTC 12493 (MRSA) | 0,004 | 5 | 85,3±0,6 | 1250 |
| S.aureus ATCC 29213 (MSSA) | 0,004 | >40 | 52,5±1,1 | Not found |
| S.aureus ATCC 43300 (MRSA) | 0,004 | >40 | 66,6±1,6 | Not found |
| S.aureus ATCC 1707 (MRSA) | 0,008 | >40 | 61,5±0,5 | Not found |

| | | | | |
|---|---|---|---|---|
| S.aureus ATCC 1717 (MRSA) | 0,008 | >40 | 72,9±1,2 | Not found |
| S.aureus ATCC 1747 (MRSA) | 0,008 | >40 | 44,9±0,7 | Not found |
| S.aureus ATCC 1761 (MRSA) | 0,016 | 40 | 90,1±0,00 | 2500 |
| S.aureus ATCC 1766 (MRSA) | 0,008 | >40 | 86,2±0,9 | Not found |
| S.aureus ATCC 1768 (MRSA) | 0,008 | >40 | 61,9±1,1 | Not found |
| S.epidermidis ATCC 35984 | 0,002 | >40 | 3,6±2,0 | Not found |
| E.coli ATCC 25922 | >40 | >40 | 0±0,00 | Not found |
| K.pneumoniae ATCC 13883 | >40 | >40 | 4,9±3,1 | Not found |
| P.mirabilis ATCC 25933 | >40 | >40 | 8,3±0,6 | Not found |
| S.enterica ATCC 14028 | >40 | >40 | 12,5±5,7 | Not found |
| P.aeruginosa ATCC 10145 | >40 | >40 | 0±0,00 | Not found |

To discard the possibility that the variability in growth inhibition observed was due to low amounts of nuclease production by the tested strains, we performed nuclease activity assays for each strain. TTprobe2 was modified with a fluorophore and a quencher, as previously described,[16] allowing for the quantification of the nuclease activity. **Figure 4** shows that all S. *aureus* strains can degrade TTprobe2 efficiently, with similar fluorescence signals, suggesting that nuclease activity is not the cause for therapeutic variability among strains. Next, we performed the MICs using Floxuridine and TOUCAN containing Floxuridine (7mFx). As indicated for 7mGe, the Floxuridine was synthesized in the phosphoramidite format to facilitate the incorporation into the TOUCAN structure, in the downstream position. Next, the MIC studies for Floxuridine and TOUCAN 7mFx were carried out under the same conditions described above. Both were able to inhibit the growth of all S. *aureus* strains tested with MIC values from 0.005 to 0.32 μg/mL for Floxuridine alone and MIC values from 0.62 to 40 μg/mL for TOUCAN 7mFx **(Table S4)**. Interestingly, efficient bacterial growth inhibition (91.5% to 94.6%) was observed for all MRSA strains tested with Floxuridine and TOUCAN 7mFx.

**Table 4.** MICs and bacterial growth inhibition in nine S. *aureus* strains by Floxuridine and TOUCAN 7mFx *(mean ± SD, n = 3).*

| Bacteria | Nucleoside Analogue (NA) | TOUCAN | TOUCAN | TOUCAN MIC / NA MIC |
|---|---|---|---|---|
| | Floxuridine MIC (µg/mL) | 7mFx MIC (µg/mL) | % Growth inhibition | TOUCAN Efficacy Ratio (TER) |
| S.aureus NCTC 12493 (MRSA) | 0,32 | 40 | 91,5±0,00 | 125 |
| S.aureus ATCC 29213 (MSSA) | 0,08 | 20 | 92,3±0,09 | 250 |
| S.aureus ATCC 43300 (MRSA) | 0,04 | 40 | 92,8±0,7 | 1000 |

| | | | | |
|---|---|---|---|---|
| S.aureus ATCC 1707 (MRSA) | 0,04 | 5 | 94,6±0,08 | 125 |
| S.aureus ATCC 1717 (MRSA) | 0,08 | 10 | 94,5±0,04 | 125 |
| S.aureus ATCC 1747 (MRSA) | 0,005 | 2,5 | 92,8±0,7 | 500 |
| S.aureus ATCC 1761 (MRSA) | 0,005 | 2,5 | 93,9±0,05 | 500 |
| S.aureus ATCC 1766 (MRSA) | 0,04 | 0,62 | 93,6±0,1 | 15,5 |
| S.aureus ATCC 1768 (MRSA) | 0,04 | 2,5 | 93,2±0,3 | 62,5 |
| S.epidermidis ATCC 35984 | 0,04 | >40 | 7,4±4,0 | Not found |
| E.coli ATCC 25922 | 20 | >40 | 0±0,00 | Not found |
| K.pneumoniae ATCC 13883 | 20 | >40 | 0±0,00 | Not found |
| P.mirabilis ATCC 25933 | 5 | >40 | 45,5±0,4 | Not found |
| S.enterica ATCC 14028 | 20 | >40 | 10,8±2,1 | Not found |
| P.aeruginosa ATCC 10145 | >40 | >40 | 7,2±1,4 | Not found |

Among all the *S. aureus* strains tested, the most susceptible to TOUCAN 7mGe and 7mFx treatment were NCTC-12493 (MRSA) and ATCC-1766 (MRSA), respectively. In order to identify the best model for further experimentation, we carried out TOUCANs dose response experiments with these two MRSA strains, along with *S. epidermidis* cultures as control bacteria **(Figure 5)**. For 7mGe, efficient inhibition was achieved with concentrations from 5 to 40 μg/mL (96%), while a partial effect was still observed with 1.25 and 2.5 μg/mL, as shown by the growth curves **(Fig 5a)** and by visual inspection after 18 hours at the corresponding concentrations **(Fig 5b)**. No growth inhibition was observed for the control bacteria, S. *epidermidis*, at all concentrations tested. Similar growth inhibitions curves and visual inspection **(Figures 5e-f)** were generated for the 7mFx, showing partial inhibition with 0.31 μg/mL and efficient inhibition with concentrations from 0.62 to 10 μg/mL. **Figures 5g-h** have shown no growth inhibition for S. *epidermidis* in the presence of 7mFx. To identify the best performing TOUCAN, we calculated the TOUCAN Efficiency Ratio (TER) by dividing the MICs values for the TOUCAN with the MICs values obtained for the nucleoside analogues. **Figure 5i** represents the bar graph of the log MIC values obtained with each treatment: Gemcitabine, 7mGe, Floxuridine and 7mFx. The calculated TERs are 1250 for 7mGe and 15.5 for 7mFx and are depicted on top of the bars in **Fig 5i**. A lower TER (15.5) indicates that therapeutic efficacy of the TOUCAN is closer to the therapeutic efficacy of the naked nucleoside analogue, suggesting a good performing TOUCAN. The raw data of dose response curves and the plate inspected by naked eye after 18 hours are shown in **Figure 6**. These results suggest that TOUCAN 7mFx has reported the most efficient growth inhibition against *S. aureus* and covers a broader spectrum of strains. Therefore, we selected TOUCAN 7mFx for further experimentation using *in vivo* infection models in mice. To carry out the animal experiments, we first optimized a mouse model of tight muscle infection based on our previous work.[16] Next, the therapeutic regime was adapted from a previous study which validates the efficacy of several antibiotics against *S. aureus*.[28] Briefly, mice were inoculated with bacteria intramuscularly, in one leg (right). The first dose of TOUCAN was administrated after 2 hours post-inoculation, followed by 3 additional injections of same concentration every 6 hours. Three groups of animals were evaluated: TOUCAN 7mFx, along with the control groups Placebo and Fluoxuridine only. A detailed description of the animal model is provided in the methods section.

**Figure 7** provides solid evidence of the therapeutic efficacy of TOUCAN 7mFx for treating S. *aureus* infections in mice. The results indicate that mice treated with Placebo (saline solution) have developed a visible bacterial infection **(Fig 7a,** black arrow), and a slightly stronger infection was observed in mice treated with Floxuridine **(Fig 7c,** black arrow). In contrast, the infected mice treated with TOUCAN 7mFx do not show any visible signs of bacterial infection, suggesting high therapeutic efficacy **(Fig 7b)**. Importantly, Floxuridine and TOUCAN 7mFx groups were treated with equivalent amounts of Floxuridine (see methods). As expected, the uninfected legs (left) for all groups did not show evidence of bacterial infection. Replicates of gross pictures for the three groups are presented in **Figure 8**. To confirm these findings, we carried out histopathological analysis and bacteria counts of the uninfected and infected legs, along with the blood analysis. The histopathological analysis showed no evidence of bacterial infection or inflammation for the uninfected legs **(Fig 7d)**. In contrast, mice treated with Placebo and Floxuridine, **Figures 7e** and **7g** respectively, have shown clear evidence of bacterial infection, inflammation, and neutrophil infiltration (black asterisk). On the other hand, minimal inflammation with residual neutrophil infiltration was seen in mice treated with TOUCAN 7mFx, suggesting that immunological clearance of the infection took place. **Fig 9** shows the histopathological images acquired with different objectives (4x, 10x and 20x). Next, we sought to quantify the number of bacteria for each treated group **(Figure 7h).** As expected, no detectable bacteria were found in the uninfected legs, while high bacteria count was detected in the Placebo and Floxuridine groups **(Figure 7h,** white and black bars, respectively). Interestingly, the number of bacteria recovered from the group treated with TOUCAN 7mFx was significantly lower, with 642 and 2949 times less bacteria when compared to Placebo and Floxuridine groups, respectively. Representative bacteria count plates are provided in **Figure 10,** where the differences of bacteria numbers among groups can be observed. Furthermore, we analyzed the percentages of neutrophils in blood samples. Neutrophils are the first line and the most efficient responders to pathogenic bacteria and therefore, they are very important for the evaluation of bacterial infections. For a thorough analysis, and to avoid biases blood from an additional group of uninfected/ untreated mice was evaluated. The uninfected/untreated group has shown a basal neutrophil population of 9,4% **(Figure 7i,** diagonal lines bar). In contrast, the blood from Placebo and Floxuridine mice have shown an important increase in the population of neutrophils, up to 27,1% and 30,3% respectively. Mice treated with TOUCAN 7mFx have reported 13,8% of neutrophils, which represents a slight increase compared to the population in uninfected mice, indicating that the bacterial infection was treated. The complete blood counts are provided as supplementary information in **Table 5.**

**Table 5.** Blood counts from uninfected/untreated and infected/treated mouse groups *(mean ± SD, n = 4).*

| Blood parameters | Units | Uninfected control | | Placebo | | TOUCAN 7mFx | | Floxuridine | |
|---|---|---|---|---|---|---|---|---|---|
| | | AVG | STD | AVG | STD | AVG | STD | AVG | STD |
| RBC | x10^12/L | 9,3 | 0,2 | 9,5 | 0,3 | 9,3 | 0,2 | 8,6 | 0,4 |
| HCT | % | 44,0 | 0,1 | 46,3 | 1,5 | 44,5 | 0,8 | 40,8 | 2,1 |
| HGB | g/dl | 13,8 | 0,3 | 14,2 | 0,5 | 14,0 | 0,3 | 12,8 | 0,6 |
| MCV | fL | 47,5 | 0,4 | 48,5 | 0,5 | 47,9 | 0,7 | 47,4 | 0,6 |
| MCH | pg | 14,8 | 0,1 | 14,9 | 0,2 | 15,0 | 0,2 | 14,8 | 0,4 |
| MCHC | g/dl | 31,3 | 0,4 | 30,7 | 0,5 | 31,4 | 0,5 | 31,5 | 0,3 |
| RET% | % | 3,0 | 0,3 | 2,2 | 2,0 | 2,8 | 0,4 | 4,5 | 0,4 |
| RET | K/µL | 279,7 | 22,3 | 298,4 | 45,3 | 257,7 | 37,4 | 384,9 | 19,6 |
| WBC | 10^9/L | 4,3 | 1,2 | 2,6 | 1,1 | 2,4 | 0,3 | 2,2 | 0,3 |
| NE% | % | 9,8 | 0,9 | 29,0 | 11,9 | 14,1 | 1,9 | 36,1 | 9,5 |
| LYM% | % | 83,6 | 0,8 | 53,6 | 6,3 | 68,6 | 6,9 | 48,9 | 10,7 |
| MON% | % | 4,0 | 0,6 | 16,0 | 5,9 | 15,7 | 9,2 | 13,5 | 12,7 |
| EO% | % | 2,3 | 1,2 | 1,3 | 0,7 | 1,3 | 0,4 | 1,0 | 0,6 |
| BAS% | % | 0,3 | 0,3 | 0,1 | 0,2 | 0,3 | 0,2 | 0,5 | 0,6 |
| NE | 10^9/L | 0,4 | 0,1 | 0,7 | 0,2 | 0,3 | 0,1 | 0,7 | 0,3 |
| LYM | 10^9/L | 3,6 | 1,0 | 1,4 | 0,7 | 1,6 | 0,4 | 1,1 | 0,4 |
| MON | 10^9/L | 0,2 | 0,1 | 0,4 | 0,3 | 0,4 | 0,2 | 0,3 | 0,2 |
| EO | 10^9/L | 0,1 | 0,1 | 0,0 | 0,0 | 0,0 | 0,0 | 0,0 | 0,0 |
| BAS | 10^9/L | 0,0 | 0,0 | 0,0 | 0,0 | 0,0 | 0,0 | 0,0 | 0,0 |
| PLT | K/µL | 743,0 | 20,9 | 820,3 | 24,2 | 630,0 | 165,6 | 599,3 | 210,3 |

Another interesting observation derived from the blood analysis was reduction of the red blood cells (RBCs), hematocrit and hemoglobin in the mice treated with Floxuridine. However, no difference was observed for the uninfected, placebo and TOUCAN 7mFx treated groups **(Figure 11).** This finding could explain the stronger infection observed in gross pictures for mice treated with Floxuridine, along with the high bacteria counts in the tissue and the highest population of neutrophils. RBCs reduction (anemia) is a marker of acute toxicity and suggests that, in part, the red blood cells were affected by the systemic administration of Floxuridine. The absence of anemia in the mice treated with TOUCAN 7mFx and the therapeutic efficacy observed in this group, suggest that most of the drug was released at the infection site with no indication of toxicity for RBCs. All these evidence in mice, suggest that TOUCAN 7mFx has the capability to treat bacterial infections caused by *S. aureus* in an efficient and safer manner. In addition, the TOUCAN technology has demonstrated the utility as a drug delivery system for nucleoside analogues, with targeted release of the drug at the infection site with limited acute toxicity in blood. Altogether, these results confirm that TOUCAN 7mFx can provide a targeted strategy to treat bacterial infections and demonstrate the great potential as a first-in-class targeted antibiotic. While promising technologies such as bacteriophages and CRISPRs still need to overcome significant limitations with their delivery and off-target effects, TOUCAN technology has demonstrated to be one step closer to the clinic. Currently, our lab is working in translating this technology for cancer treatments and using other types of drug moieties, such as doxorubicin and mitomycin C.

To demonstrate the applicability of TOUCAN technology for other important pathogenic bacteria, we have treated two gram negative bacteria *(E. coli* and *P. mirabilis)* with the TOUCAN 7mFx and TOUCAN 7mFx spiked with the specific nuclease (MN), along with Floxuridine alone. Figure 12 shows the therapeutic utility of TOUCAN for gram negative bacteria. These results indicate that if a given nuclease is successfully targeted by an oligonucleotide substrate, a therapeutic strategy can be developed using TOUCAN generic platform. Next, we have used the same spiking strategy to demonstrate the applicability of TOUCAN technology in mammalian cells, more specifically in cancer cells. First, we checked the viability of pancreatic cancer cells in 72 h cultures using a fluorescent assay. The cultures were treated with either Gemcitabine or TOUCAN 7mGe spiked with MN at concentrations indicated in the Figure 13, along with untreated cells as control. Figure 13 indicates that both treatments were efficient in killing pancreatic cancer cells. To confirm these preliminary results, we have also tested breast cancer cells under the same conditions as previously described. As indicate in Figure 14, after 72 h of treatment a clear therapeutic effect for Gemcitabine and slightly higher for TOUCAN 7mGe spike with MN was observed in breast cancer cells. Altogether, these results confirm the generic applicability of TOUCAN technology to several human conditions, and more specifically in the treatment of cancer cells, gram positive and gram-negative bacteria, and any other indication where a nuclease can be used as biomarker.

We have also explored the possibility of delivering other therapeutic molecules than nucleoside analogues. This strategy could provide targeted delivery of a broad range of small molecule drugs currently used in the clinic. To prove this strategy, we have synthetized 7mDx (UUUUTTDx), an oligonucleotide consisting of a resistant moiety and a specific moiety as previously described, modified at the 3'-end with Doxorubicin. To carry out the synthesis of this TOUCAN, we have first synthetized Doxorubicin in phosphoramidite format. Next, we evaluated the therapeutic activity of the TOUCAN 7mDx construct in breast cancer cells. We treated the cells with Doxorubicin alone, 7mDx alone, 7mDx spiked with MN, along with untreated cells as control. Figure 15 shows a remarkable therapeutic efficacy of the 7mDx spiked with MN, similar to doxorubicin alone, suggesting that a targeted strategy for Doxorubicin based on TOUCAN technology can be developed. Importantly, 7mDx alone did not show a significant effect on viability, indicating that in the absence of the specific nuclease, no toxicity is expected with this approach. These results confirm the versatility of this technology and the use of small molecules that can be synthesized in phosphoramidite format, offering a great platform for developing cancer targeted treatments.

Therefore, a first aspect of the invention refers to a drug delivery system which comprises a polynucleotide sequence susceptible of being cleaved by a specific nuclease derived from a bacterium or a mammalian cell, wherein the polynucleotide sequence, preferably from (upstream to downstream) 5' to 3' or also preferably from 3' to 5', comprises i) an oligonucleotide sequence of RNase-resistant modified RNA bases and/or nuclease-resistant DNA bases, acting as a resistant moiety, directly linked to ii) a polynucleotide sequence susceptible of being cleaved by a specific nuclease derived from a bacterium or a mammalian cell which is in turn directly linked to iii) a pharmacological active ingredient, wherein the polynucleotide sequence when cleaved at the cleaving site, releases the pharmacological active ingredient.

In the context of the present invention, the term "resistant moiety" shall be understood as RNase-resistant modified RNA bases, or nuclease-resistant DNA bases. A resistant moiety may encompass nucleotides with a covalently modified backbone, base and/or sugar that confers resistance for RNase and/or Dases. For example, modified nucleotides include nucleotides having sugars which are covalently attached to low molecular weight organic groups other than a hydroxyl group at the 3' position and other than a phosphate group at the 5' position. Thus modified nucleotides may also include 2' substituted sugars such as 2'-O-methyl-; 2-O-alkyl; 2-O-allyl; 2'-S-alkyl; 2'-S-allyl; 2'-fluoro-; 2'-halo or 2- azido-ribose, LNA, UNA, FANA, ANA carbocyclic sugar analogues a-anomeric sugars; epimeric sugars such as arabinose, xyloses, pyranose sugars, furanose sugars, and sedoheptulose. In certain embodiments, the resistant moiety includes, but is not limited to, 2'-O-methyl RNA, 2'-methoxyethoxy RNA, 2'-O-allyl RNA, 2'-O-pentyl RNA, and 2'-O-butyl RNA. In certain embodiments, the resistant moiety is an RNA-2'-O-methyl RNA oligonucleotide. It is noted that modified nucleotides are known in the art and include, by example and not by way of limitation, alkylated purines and/or pyrimidines; acylated purines and/or pyrimidines; or other heterocycles. These classes of pyrimidines and purines are known in the art and include, pseudoisocytosine; N4, N4-ethanocytosine; 8-hydroxy-N6-methyladenine; 4- acetylcytosine, 5-(carboxyhydroxylmethyl) uracil; 5-fluorouracil; 5-bromouracil; 5- carboxymethylaminomethyl-2-thiouracil; 5-carboxymethylaminomethyl uracil; dihydrouracil; inosine; N6-isopentyl-adenine; I-methyladenine; 1-methylpseudouracil; 1- methylguanine; 2,2-dimethylguanine; 2-methyladenine; 2-methylguanine; 3-methylcytosine; 5-methylcytosine; N6-methyladenine; 7-methylguanine; 5-methylaminomethyl uracil; 5- methoxy amino methyl-2-thiouracil; β-D-mannosylqueosine; 5- methoxycarbonylmethyluracil; 5-methoxyuracil; 2-methylthio-N6-isopentenyladenine; uracil-5-oxyacetic acid methyl ester; psueouracil; 2-thiocytosine; 5-methyl-2 thiouracil, 2- thiouracil; 4-thiouracil; 5-methyluracil; N-uracil-5-oxyacetic acid methylester; uracil 5- oxyacetic acid; queosine; 2-thiocytosine; 5-propyluracil; 5-propylcytosine; 5-ethyluracil; 5- ethylcytosine; 5-butyluracil; 5-pentyluracil; 5-pentylcytosine; and 2,6,-diaminopurine; methylpsuedouracil; 1-methylguanine; 1-methylcytosine.

In the context of the present invention, the term "pharmacological active ingredient" shall be understood as any small molecule that can furnish pharmacological activity or to otherwise have direct effect in the cure, mitigation, treatment or prevention of disease, or to have direct effect in restoring, correcting or modifying physiological functions in human beings and that can be coupled to a polynucleotide sequence.

In the context of the present invention, the term "cleaving site" or "polynucleotide sequence susceptible of being cleaved" shall be understood as the region where the biorecognition event between the oligonucleotide and the nuclease takes place and where a given nuclease cleaves the oligonucleotide and releases the drug moiety.

The drug delivery system of the invention thus comprises polynucleotide sequences (also referred to as "Substrate oligonucleotides") that are substrates for nuclease (e.g., endoribonuclease) enzymes. Substrate oligonucleotides of the invention comprise mainly two different parts: 1) RNase-resistant modified RNA bases and/or nuclease-resistant DNA bases, and 2) one or more nuclease-cleavable bases, e.g., RNA bases, some or all of which function as scissile linkages, wherein the nuclease-cleavable bases are directly linked to a pharmacological active ingredient. Synthetic oligonucleotide RNA-DNA chimeras wherein the internal RNA bonds function as a scissile linkage are described in US Patent Nos. 6,773,885 and 7,803,536. The resistant moiety and the pharmacological active ingredient are separated by one of these nuclease-cleavable residues, e.g., RNA base. Such residues serve as a cleavage domain for ribonucleases.

The substrate oligonucleotides of the invention can be synthesized using conventional phosphoramidite synthesis using techniques which are known in the art. Linkages between nucleotides may use alternative linking molecules. For example, linking groups of the formula P(O)S, (thioate); P(S)S, (dithioate); P(O)NR'2; P(O)R'; P(O)OR6; CO; or CONR*2 wherein R is H (or a salt) or alkyl (1-12C) and R6 is alkyl (1-9C) is joined to adjacent nucleotides through -O- or -S-. The oligonucleotide substrates (resistant and specific region) preferably will be coupled to the drug moiety by phosphoramidite synthesis. To do so, the drug molecule should be synthesized as a phosphoramidite building block. Moreover, other coupling chemistries known in the art can be used for the incorporation of the drug moiety into the polynucleotide sequence. Functional groups for the oligonucleotide coupling include but are not limited to amine, hydroxyl, thiol, maleimide, hydrazide, aldehyde, phosphate, phophorothioate, azide, alkyne, etc.

In a preferred embodiment of the first aspect of the invention, the polynucleotide sequence, from (upstream to downstream) 5' to 3' or alternatively from 3' to 5', consists of i) an oligonucleotide sequence of RNase-resistant modified RNA bases and/or nuclease-resistant DNA bases, acting as a resistant moiety, preferably directly, linked to ii) a polynucleotide sequence susceptible of being cleaved by a specific nuclease derived from a bacterium or a mammalian cell, wherein the polynucleotide sequence susceptible of being cleaved by a specific nuclease is, preferably directly, linked to a pharmacological active ingredient, and wherein the polynucleotide sequence when cleaved at the cleaving site, releases the pharmacological active ingredient.

In another preferred embodiment of the first aspect of the invention, the oligonucleotide sequence of RNase-resistant modified RNA bases, or nuclease-resistant DNA bases, acting as a resistant moiety, comprises or consists of 2'-O-Methyl nucleosides, (-mC- -mU- -mA- or -mG-), LNA ($_LC$ $_LU$ $_LA$ $_LG$) Other possible oligonucleotide sequences of RNase-resistant modified RNA bases, or nuclease-resistant DNA bases can be selected from the list consisting of 2'-Fluoro, FANA, ANA, etc.

In another preferred embodiment of the first aspect of the invention, the polynucleotide sequence is susceptible of being cleaved by a specific nuclease derived from bacteria listed in Table 6.

**Table 6.** List of bacteria with potential nucleases as biomarker

| Bacteria | Nuclease | Type of nuclease | Substrate | Cofactors | Function in pathogenesis | Reference |
|---|---|---|---|---|---|---|
| *Staphylococcus aureus* | Nuc1 | Extracellular | ss/ds-linear DNA, circular DNA and RNA | $Ca^{+2}$, $Mg^{+2}$ | Resistance against NET mediated killing | Tucker et al., 1978 |
| | Nuc2 | Membrane-associated | Both DNA and RNA | $Ca^{+2}$, $Mg^{+2}$ | Biofilm dispersal | Hu et al., 2013 |
| *Streptococcus pyogenes* | SpnA | Cell-wall associated | ss/ds-linear DNA, circular DNA and RNA | $Ca^{+2}$, $Mg^{+2}$ | Resistance against NET mediated killing | Chang et al., 2011 |
| | MF/SpeF/SdaB/Spd/DNaseB | Extracellular | ss/ds-linear DNA, circular DNA and RNA | $Ca^{+2}$, $Mg^{+2}$ | Mitogenicity | Iwasaki et al., 1993 |
| | MF2/Spd1/SdaC/DNaseC | Extracellular | ss/ds-linear DNA, circular DNA and RNA | $Ca^{+2}$, $Mg^{+2}$ | Mitogenicity | Broudy et al., 2002 |
| | SdaD/DNaseD | Extracellular | DNA | NA | Resistance to phagocytosis | Podbielski et al., 1996 |
| | MF3/Spd13 | Extracellular | ss/ds-linear DNA, circular DNA and RNA | $Ca^{+2}$, $Mg^{+2}$ | Mitogenicity | Ferretti et al., 2001 |
| | Sda1/SdaD2 | Extracellular | DNA | $Ca^{+2}$, $Mg^{+2}$ | Resistance to NETs | Aziz et al., 2004 |
| | MF4 | Extracellular | NA | NA | Mitogenicity | Aziz et al., 2004 |
| *Streptococcus agalactiae* | NucA | Extracellular | ss/ds-linear DNA, circular DNA and RNA | $Mg^{+2}$ | Resistance to NETs | Derré-Bobillot et al., 2013 |
| *Streptococcus pneumoniae* | EndA | Membrane-associated | ss/ds-linear DNA, circular DNA | $Mg^{+2}$ | Degradation of NETs; | Moon et al., 2011 |

| Bacteria | Nuclease | Type of nuclease | Substrate | Cofactors | Function in pathogenesis | Reference |
|---|---|---|---|---|---|---|
| Escherichia coli | EndoI | Periplasm | dsDNA | Mg$^{+2}$ | Prevention of uptake of foreign DNA | Jekel and Wackernagel, 1995 |
| | ColicinE2 | Extracellular | ss/dsDNA, circular DNA and RNA | Zn$^{+2}$ | Cytotoxicity | Schaller and Nomura, 1976 |
| | ColicinE7 | Extracellular | dsDNA and RNA | Zn$^{+2}$, Mg$^{+2}$, Ni$^{+2}$, Mn$^{+2}$ | Cytotoxicity | Ko et al., 1999; Sui et al., 2002 |
| | ColicinE9 | Extracellular | ss/dsDNA, circular DNA and RNA | Mg$^{+2}$, Ni$^{+2}$ | Cytotoxicity | Chak et al., 1991 |
| Klebsiella pneumoniae | RecBCD | Extracellular | dsDNA | Mg$^{+2}$ | Prevention of uptake of foreign DNA | Wilkinson M et al., 2016 |
| Serratia marcescens | Sm nuclease | Extracellular | ss/ds-linear DNA, circular DNA and RNA | Mg$^{+2}$ | Prevention of uptake of foreign DNA | Ball et al., 1987; Suh et al., 1995 |
| Salmonella Typhimirium | Endonuclease V | Extracellular | DNA | Mg$^{+2}$, Mn$^{+2}$ | DNA repair | Feng H et al., 2005 |
| Stenotrephomonas maltophilia | RNaseG | Extracellular | RNA | Mg$^{+2}$ | Cellular response to stress | Bernardini A et al., 2017 |
| Pseudomonas aeruginosa | EddB | Extracellular | Genomic DNA | Ca$^{+2}$, Mg$^{+2}$ | Nutrition | Mulcahy et al., 2010 |
| | EndA | Extracellular | Genomic DNA | NA | Biofilm dispersion | Cherny et al.. 2019 |
| | AP41 | Extracellular | Circular DNA | Mg$^{+2}$, Mn$^{+2}$ | Cytotoxicity | Sano et al., 1993 |
| | S1 | Extracellular | Circular DNA | Mn$^{+2}$ | Cytotoxicity | Sano et al., 1993 |
| | 52 | Extracellular | Circular DNA | Mn$^{+2}$ | Cytotoxicity | Sano et al., 1993 |
| | S3 | Extracellular | Linear DNA | NA | Cytotoxicity | Duport et al., 1995 |
| Acinetobacter baumannii | NucAB | Membrane-associated | DNA | NA | NA | Garg N et al., 2016 |
| Moraxella catarrhalis | NucM | Extracellular | Both DNA and RNA | Mg$^{+2}$ | Cell entry, cell aggregation, and biofilm formation | Tan A et al., 2019 |

| Bacteria | Nuclease | Type of nuclease | Substrate | Cofactors | Function in pathogenesis | Reference |
|---|---|---|---|---|---|---|
| *Neisseria gonorrhoeae* | Nuc | Periplasm | dsDNA and ssDNA | $Ca^{+2}$, $Mg^{+2}$ | Biofilm structural organisation | Steichen et al., 2011 |
| *Haemophilus influenzae* | Nuc | Extracellular | ss/ds-linear DNA and circular DNA | $Ca^{+2}$, $Mg^{+2}$ | Biofilm remodelling and dispersal | Cho et al., 2015 |
| *Mycobacterium tuberculosis* | Rv0888 | Extracellular | ss/ds-linear DNA, circular DNA and RNA | $Ca^{+2}$, $Mn^{+2}$ | Biofilm formation, utilization of extracellular DNA as a nutrient, and degradation of NETs | Dang et al., 2016 |
| *Helicobacter pylori* | NucT | Membrane-associated | ss/ds-linear DNA, circular DNA and RNA | Not required | Natural transformation; utilization of DNA as a source of purines | Liechti and Goldberg, 2013; O'Rourke et al., 2004 |
| *Campylobacter jejuni* | Dns | Extracellular | dsDNA | $Mg^{+2}$ | Prevention of uptake of foreign DNA | Gaasbeek et al., 2009 |
| *Vibrio cholerae* | Dns | Extracellular | ss/ds-linear DNA, circular DNA | $Mg^{+2}$ | Biofilm architecture and dispersal; prevention of foreign DNA uptake; nutrition | Altermark et al., 2007 |
| | Xds | Extracellular | Linear DNA | $Mg^{+2}$ | Biofilm architecture and dispersal | Newland et al., 1985 |
| *Mycoplasma pneumoniae* | Mpn133 | Membrane-associated | ss/ds-linear DNA, circular DNA and RNA | $Ca^{+2}$ | Reduce cell viability | Somarajan et al., 2010 |
| *Mycoplasma penetrans* | Endonuclease P40 | Membrane-associated | ss/ds-linear DNA, circular DNA and RNA | $Ca^{+2}$, $Mg^{+2}$ | Induction of apoptosis | Bendjennat et al., 1997 |

In another preferred embodiment of the first aspect of the invention, the polynucleotide sequence susceptible of being cleaved by a specific nuclease derived from mammalian cells, is cleaved by any of the following type of cancer cells: Bladder Cancer cells, Breast Cancer cells, Colon and Rectal Cancer cells, Endometrial Cancer cells, Kidney Cancer cells, Leukemia cells, Liver Cancer cells, Lung Cancer cells, Melanoma cells, Non-Hodgkin Lymphoma cells, Pancreatic Cancer cells, Prostate Cancer cells, and Thyroid Cancer cells, among others.

| Nuclease | Type of Cancer | References |
| --- | --- | --- |
| FEN1 | gastric, breast, prostate, lung, pancreatic, brain, uterine, colon, and kidney cancer | He et al., 2016<br>Singh et al., 2008<br>K. Wang, Xie, & Chen, 2014<br>Abdel-Fatah et al., 2014 |
| APE1 (Human apurinic / apyrimidinic endonuclease1) | non-small cell lung-cancer | D. Wang et al. 2009<br>Sevilya et al., 2015 |
| | lung cancer | Moore et al., 2000; Al-Attar et al., 2010 |
| | ovarian cancer | Woo et al., 2014; AlMutairi et al., 2015 |
| | breast cancer | Madhusudan, 2013 |
| | gastro-esophageal cancer, ovarian cancer and pancreatico-biliary | Wei et al.,2017 |
| | cancer | Kelley et al., 2001 |
| | gastric cancer | Di Maso et al., 2015, Pascut et al., 2019) |
| | prostate cancer | |
| | hepatocellular carcinoma | Shin et al., 2015 |
| | bladder cancer | |
| | colorectal cancer | Koukourakis et al., 2001 |
| | head and neck cancer melanoma | Abbotts et al., 2014 |
| XPF/XPG | non-small cell lung | (Olaussen et al., 2006 |
| | cancer | McNeil & Melton, 2012 |
| | melanoma | Shirota et al., 2001 |
| | colorectal cancer | Walsh et al., 2008 |
| | ovarian cancer | Doherty & Madhusudan, 2015 |
| | gastric cancer | |
| SND1 (Staphylococcal nuclease domain containing-1) | human hepatocellular | Santhekadur et al., 2012 |
| | carcinoma | Kuruma et al., 2009 |
| | prostate cancer | Tsuchiya et al., 2007 |
| | colon cancer | |
| DNAse I | gastric carcinoma | Tsutsumi et al., 1998 |
| | colorectal carcinoma | Tsutsumi et al., 2000 |
| | lung cancer | Yasuda et al., 2005 |
| RNAseL | prostate cancer | Wang et al., 2017; Nupponen et al., 2004 |
| | uterine cervix cancer | |
| | HNSCC breast cancer | Madsen et al., 2008 |
| RNase 1 | pancreatic adenocarcinoma | Nakata, 2014 |

In another preferred embodiment of the first aspect of the invention, the pharmacological active ingredient is selected from the list that includes but are not limited to:

i) ALKYLATING AGENTS: Cyclophosphamide, Mechlorethamine, Ifosfamide, Melphalan, Chlorambucil, Streptozocin, Carmustine, Lomustine, Busulfan, Dacarbazine, Temozolomide, Thiotepa, Altretamine, Treosulfan, Mannosulfan, Trofosfamide, Triaziquone, Carboquone, Nimustine, Ranimustine,

**ii)** KINASE INHIBITOR DRUGS: Imatinib, Gefinitib, Toceranib, Erlotinib, Lapatinib, Sunitinib, Sorafenib, Nilotinib, Crizotinib, Ceritinib, Alectinib, Brigatinib, Lorlatinib, Acalabrutinib, Ibrutinib, Zanubrutinib, Midostaurin, Gilteritinib, Quizartinib Crenolanib Ruxolitinib, Pertuzumab, Trastuzumab, Neratinib, Lapatinib, Trastuzumab emtansine, Dacomitinib, Gefitinib, Panitumumab, Erlotinib, Cetuximab, Osimertinib, Vandetanib, Necitumumab, Dacomitinib, Ponatinib, Bevacizumab, Aflibercept, Cabozantinib, Axitinib, Regorafenib, Ramucirumab, Lenvatinib, Vemurafenib, Dabrafenib, Abemaciclib, Palbociclib, Ribociclib, Seliciclib, Cobimetinib, Trametinib, Larotrectinib

**iii)** VINCA ALKALOIDS: Vinblastine, Vinorelbine, Vincristine, Vindesine,

**iv)** ANTHRACYCLINES: Doxorubicin, Danorubicin (Daunomycin), Epirubicin, Idarubicin, Valrubicin

**v)** ANTIMETABOLITES: Gemcitabine, Floxuridine, Arabinosylcytosine (Cytarabine), Azacitidine, Capecitabine, Clofarabine, Cladribine, Decitabine, Fludarabine, Fluorouracil, Hydroxyurea, Folinic acid (Leucovorin), Mercaptopurine, Nelarabine, Pentostatin, Procarbazine, Thioguanine, Trifluridine

**vi)** AROMATASE INHIBITORS: Letrozole, Exemestane, Anastrozole, Aminoglutethimide,

**vii)** TOPOISOMERASE INHIBITORS: Camptothecin, Etoposide, Irinotecan, Mitoxantrone, Topotecan,

**viii)** mTOR INHIBITORS: Temsirolimus, Everolimus, Ridaforolimus, Dactolisib, Sapanisertib

**ix)** RETINOIDS: Tretinoin, Alitretinoin, Bexarotene, Isotretinoin

**x)** HISTONE DEACETYLASE INHIBITORS: Belinostat, Panobinostat, Romidepsin, Vorinostat

**xi)** PARP INHIBITORS: Niraparib, Rucaparib, Olaparib, Talazoparib

Preferably, the pharmacological active ingredient is selected from the list consisting of floxuridine, doxorubicin and gemcitabine, more preferably, the pharmacological active ingredient is selected from the list consisting of floxuridine, and doxorubicin.

In another preferred embodiment of the first aspect of the invention, the polynucleotide sequence is selected from the group consisting of mUmUmUmUTT -Drug (i.e. Gemcitabine - Floxuridine) for S. aureus, or mUmUmUmU-U-Drug (e.i.Resdisivir) for coronavirus.

In another preferred embodiment of the first aspect of the invention, the polynucleotide sequence is selected from the group consisting of mUmUmUmUfAfAfA- Drug (i.e. Gemcitabine - Doxorubicin); for pancreatic/breast cancer.

In another preferred embodiment of the first aspect of the invention, the polynucleotide sequences of the invention are at least 3 nucleotides in length, such as 3-30 nucleotides in length. In certain specific embodiments, the nucleic acids of the invention are 3-20, 3-15, 3-10, 7-20, 7-15, 7-10, 5-50 or 10-50 nucleotides in length. Preferably, the polynucleotide sequence comprises i) an oligonucleotide sequence of RNase-resistant modified RNA bases and/or nuclease-resistant DNA bases, having 2-24 nucleotides in length, directly linked to ii) a polynucleotide sequence susceptible of being cleavage by a specific nuclease derived from a bacterium or a mammalian cell, having 1-5 nucleotides in length. Preferably, the polynucleotide sequence comprises i) an oligonucleotide sequence of RNase-resistant modified RNA bases and/or nuclease-resistant DNA bases, having 4 nucleotides in length, directly linked to ii) a polynucleotide sequence susceptible of being cleavage by a specific nuclease derived from a bacterium or a mammalian cell, having 2 nucleotides in length.

In another preferred embodiment of the first aspect of the invention, the polynucleotide sequence susceptible of being cleaved by a specific nuclease derived from a bacterium or a mammalian cell is linked to the pharmacological active ingredient by using phosphoamidate synthesis or other coupling chemistries known in the art can be used for the incorporation of the drug moiety into the polynucleotide sequence, such as: amino-, carboxylic, azide, thiol-, ester-, maleimide, click chemistry, etc.

In another preferred embodiment of the first aspect of the invention, the polynucleotide sequence comprises i) an oligonucleotide sequence of RNase-resistant modified RNA bases and/or nuclease-resistant DNA bases, having 4 nucleotides in length, directly linked to ii) a polynucleotide sequence susceptible of being cleaved by a specific nuclease derived from a bacterium or a mammalian cell, having 2 nucleotides in length, wherein the polynucleotide sequence susceptible of being cleaved by a specific nuclease derived from a bacterium or a mammalian cell is linked to the pharmacological active ingredient by phosphoamidate synthesis or other coupling chemistries known in the art , and wherein the oligonucleotide sequence of RNase-resistant modified RNA bases, or nuclease-resistant DNA bases, acting as a resistant moiety comprises or consists of 2'-O-Methyl nucleosides (-mC- -mU- -mA- or - mG-).

In another preferred embodiment of the first aspect of the invention, the pharmacological active ingredient is a bacteriostatic or bactericide compound and the polynucleotide sequence is susceptible of being cleavage by a specific nuclease derived from a bacterium.

In another preferred embodiment of the first aspect of the invention, the pharmacological active ingredient is selected from the list consisting of a floxuridine, doxorubicin and gemcitabine and the polynucleotide sequence is susceptible of being cleaved by a specific nuclease derived from a bacterium.

In another preferred embodiment of the first aspect of the invention, the pharmacological active ingredient is selected from the list consisting of a floxuridine, doxorubicin and gemcitabine and the polynucleotide sequence is susceptible of being cleaved by a specific nuclease derived from a mammalian cell. In another preferred embodiment of the first aspect of the invention, the polynucleotide sequence is selected from the group consisting of of mUmUmUmUTT -Drug (e.i.Gemcitabine -Floxuridine) for S. aureus, and mUmUmUmU-U- Drug (e.i.Resdisivir) for coronavirus,

In another preferred embodiment of the first aspect of the invention, the polynucleotide sequence is selected from the group consisting of mUmUmUmUfAfAfA- Drug (e.i. Gemcitabine - Doxorubicin) for pancreatic cancer, leukemic etc....

In yet another preferred embodiment of the first aspect of the invention, the polynucleotide sequence comprises 0- 50% purines (or any value in between). In certain embodiments the oligonucleotide comprises 100% pyrimidines. It should be, at any rate, noted that the specific sequence of the oligonucleotide is not critical. Certain combinations of purines and pyrimidines are susceptible to bacterial endonucleases, while resisting mammalian nucleases. Endonucleases are enzymes that cleave the phosphodiester bond within a polynucleotide chain, in contrast to exonucleases, which cleave phosphodiester bonds at the end of a polynucleotide chain. These bacterial nucleases are not sequence-specific like restriction enzymes, which typically require a recognition site and a cleavage pattern. Some endonucleases cleave single-stranded nucleic acid molecules, while others cleave doublestranded nucleic acid molecules.

In a second aspect of the invention, the drug delivery system of the invention as described above (in the first aspect of the invention) can be prepared as pharmaceutically-acceptable compositions. In certain embodiments, the drug delivery system of the invention is administered so as to result in treating, ameliorating or reducing a microbial infection in a subject in need thereof. The amount administered will vary depending on various factors including, but not limited to, the composition chosen, the particular disease, the weight, the physical condition, and the age of the subject. Such factors can be readily determined by the clinician employing animal models or other test systems, which are well known to the art.

Bacterial or microbial diseases susceptible of being treated with the pharmaceutically-acceptable compositions of the present invention are any GRAM positive or GRAM negative bacteria such as those selected from the group consisting of but not limited to: Acinetobacter baumannii, Actinomyces species, Aeromonas species, Bacillus species, Bacteroides species, Bartonella species, Bordetella pertussis, Borrelia species, Brucella species, Burkholderia species, Campylobacter species, Chlamydia species, Citrobacter species, Clostridium species, Corynebacterium species, Coxiella burnetiid, Enterobacter species, Enterococcus species, Escherichia coli, Francisella tularensis, Fusobacterium necrophorum, Gardnerella vaginalis, Haemophilus species, Helicobacter Pylori, Klebsiella species, Legionella species, Leptospira species, Listeria monocytogenes, Moraxella catarrhalis, Morganella species, Mycobacterium species, Mycoplasma species, Neisseria species, Nocardia species, Pasteurella multocida, Peptostreptococcus species, Porphyromonas species, Propionibacterium species, Proteus species, Providencia species, Pseudomonas aeruginosa, Rickettsia species, Salmonella species, Serratia marcescens, Shigella species, Staphylococcus species, Stenotrophomonas maltophilia, Streptococcus species, Treponema pallidum, Ureaplasma species, Vibrio species, Yersinia species.

In a third aspect of the invention, the drug delivery system of the invention is administered so as to result in treating, ameliorating or reducing a tumour or a cancer disease in a subject in need thereof. The amount administered will vary depending on various factors including, but not limited to, the composition chosen, the particular disease, the weight, the physical condition, and the age of the subject. Such factors can be readily determined by the clinician employing animal models or other test systems, which are well known to the art.

Tumours or cancer diseases susceptible of being treated with the pharmaceutically-acceptable compositions of the present invention are selected from the group consisting of Bladder Cancer, Breast Cancer, Colon and Rectal Cancer, Endometrial Cancer, Kidney Cancer, Leukemia, Liver Cancer, Lung Cancer, Melanoma, Non-Hodgkin Lymphoma, Pancreatic Cancer, Prostate Cancer, Thyroid Cancer, among others.

Pharmaceutical formulations, dosages and routes of administration for nucleic acids useful in the second aspect of the present invention are generally known in the art. The present invention envisions detecting a microbial infection in a mammal by the administration of a drug delivery system of the invention. Both local and systemic administration is contemplated.

One or more suitable unit dosage forms of the drug delivery system of the invention can be administered by a variety of routes including parenteral, including by intravenous and intramuscular routes, as well as by direct injection into the diseased tissue. The formulations may, where appropriate, be conveniently presented in discrete unit dosage forms and may be prepared by any of the methods well known to pharmacy. Such methods may include the step of bringing into association the probe with liquid carriers, solid matrices, semi-solid carriers, finely divided solid carriers or combinations thereof, and then, if necessary, introducing or shaping the product into the desired delivery system.

When the probes of the invention are prepared for administration, in certain embodiments they are combined with a pharmaceutically acceptable carrier, diluent or excipient to form a pharmaceutical formulation, or unit dosage form. The total active ingredient (i.e., probe) in such formulations include from 0.1 to 99.9% by weight of the formulation. A "pharmaceutically acceptable" is a carrier, diluent, excipient, and/or salt that is compatible with the other ingredients of the formulation, and not deleterious to the recipient thereof. The active ingredient for administration may be present as a powder or as granules, as a solution, a suspension or an emulsion.

In a third aspect of the invention, the drug delivery system of the invention as described above (in the first aspect of the invention) can be prepared as pharmaceutically-acceptable compositions. In certain embodiments, the drug delivery system of the invention is administered so as to result in the treatment of a microbial infection to a subject in need thereof. The amount administered will vary depending on various factors including, but not limited to, the composition chosen, the particular disease, the weight, the physical condition, and the age of the subject. Such factors can be readily determined by the clinician employing animal models or other test systems, which are well known to the art.

Pharmaceutical formulations, dosages and routes of administration for nucleic acids are generally known in the art. The present invention envisions detecting a microbial infection in a mammal by the administration of a drug delivery system of the invention. Both local and systemic administration is contemplated.

One or more suitable unit dosage forms of the probe of the invention can be administered by a variety of routes including parenteral, including by intravenous and intramuscular routes, as well as by direct injection into the diseased tissue. The formulations may, where appropriate, be conveniently presented in discrete unit dosage forms and may be prepared by any of the methods well known to pharmacy. Such methods may include the step of bringing into association the probe with liquid carriers, solid matrices, semi-solid carriers, finely divided solid carriers or combinations thereof, and then, if necessary, introducing or shaping the product into the desired delivery system.

When the probes of the invention are prepared for administration, in certain embodiments they are combined with a pharmaceutically acceptable carrier, diluent or excipient to form a pharmaceutical formulation, or unit dosage form. The total active ingredient (i.e., probe) in such formulations include from 0.1 to 99.9% by weight of the formulation. A "pharmaceutically acceptable" is a carrier, diluent, excipient, and/or salt that is compatible with the other ingredients of the formulation, and not deleterious to the recipient thereof. The active ingredient for administration may be present as a powder or as granules, as a solution, a suspension or an emulsion.

The following examples provide further insight into the results already discussed in the detailed description of the invention.

## Examples

Materials and Methods

**Bacteria strains.** *S. aureus* NCTC 12493 (MRSA), *S. aureus* ATCC 29213 (MSSA), *S. aureus* ATCC 43300 (MRSA), *S. aureus* ATCC 1707 (MRSA), *S. aureus* ATCC 1717 (MRSA), *S. aureus* ATCC 1747 (MRSA), *S. aureus* ATCC 1761 (MRSA), *S. aureus* ATCC 1766 (MRSA), *S. aureus* ATCC 1768 (MRSA), *S. epidermidis* ATCC 35984, *E. coli* ATCC 25922, *K. pneumoniae* ATCC 13883, *P. mirabilis* ATCC 25933, *S. enterica* ATCC 14028 and *P. aeruginosa* ATCC 10145 strains were used in this study. Bacteria were initially inoculated onto tryptone soy agar with 5% defibrinated sheep blood (Thermo Fisher Scientific, Inc., Waltham, MA, USA) and incubated at 37°C for 24 hours. Then, single colonies of each strain were transferred to tryptone soy broth (Thermo Fisher Scientific) for the nuclease activity assay or antimicrobial susceptibility testing, respectively.

**Nucleoside Analogues (NA).** All nucleoside analogues: Gemcitabine hydrochloride, Floxuridine, Cytarabine, Vidarabine, Idoxuridine, and Trifluridine were purchased from Interchim, France. 1mg/mL stocks solutions of nucleoside analogues were prepared according to manufacturer's recommendations.

**Phosphoramidite synthesis.** The synthesis of Gemcitabine and Floxuridine phosphoramidite is schematically described in **the detailed description of the invention and figures.**

*Gemcitabine*: Gemcitabine·HCl (1) was N-benzoylated at the nitrogen base pair in a two steps procedure using trimethylsilyl chloride (TMSCl) and benzoyl chloride with a yield of 83% (2) followed by the addition of 4,4'-dimethoxytrityl-chloride (DMTrCl) with a yield of 65% (3). Next, 2-cyanoethyl-N,N-diiso-propylchlorophosphoramidite was added to complete the synthesis (4), at a final yield of 60%.

*Floxuridine*: Floxuridine (1) was selectively protected with DMTrCl at the 5' position, 73% (2) followed by the addition of 2-cyanoethyl-N,N-diiso-propylchlorophosphoramidite in 1,8-diazabicyclo[5.4.0]undec-7-ene (DBU), with a yield of 62% (3).

**Oligonucleotide synthesis.** All oligonucleotides were synthesized by the standard method of solid-phase phosphoramidite chemistry, followed by high-performance liquid chromatography (HPLC) purification. Next, successful oligonucleotide synthesis was confirmed with matrix-assisted laser desorption ionization mass spectrometry (MALDI-MS). The purity of the oligonucleotides is typically greater than 95%.

**Nuclease activity assay.** Nuclease activity of different *S. aureus* strains was evaluated using the TTprobe and TTprobe 2, along with the *S. epidermidis* as negative control and Micrococcal Nuclease (MN) as positive control. Single colonies of each bacteria strain isolated on tryptone soy agar were transferred to 50 mL tryptone soy broth and incubated at 37°C for 24 hours with 200 rpm shaking. After the incubation period, 100 µL volumes of liquid cultures were transferred to a fresh 50 mL tryptone soy broth (1/500 dilution) and incubated under the same conditions for 12 hours. Next, cultures were centrifuged at 4500 g for 30 minutes and the supernatants were used for the nuclease activity assay. Briefly, 8 µL of supernatants were mixed with 1 µL PBS +/+ and 1 µL TT probe. The reactions were incubated at 37°C for 1 hour. Then, 290 µL of 10 mM EDTA in PBS -/- solution were added to stop the reaction and the final solution was divided into triplicate wells (95 µL each), ina black flat-bottom polystyrene plates (Thermo Scientific F96). Fluorescence intensity was acquired at 485/20 excitation and 528/20 emission filter pair using Cytation 1 Cell Imaging Multimode Reader (Biotek Instruments Inc., USA).

**Antimicrobial susceptibility testing.** Minimal inhibitory concentrations (MIC) of all nucleoside analogues and TOUCANs against each bacteria strain were determined using broth microdilution method. A single colony obtained from overnight culture on tryptone soy agar with 5% defibrinated sheep blood was inoculated in cation-adjusted Mueller Hinton Broth and adjusted to $10^6$ CFU/mL. TOUCANs were prepared in PBS+/+ (treatment) and serial dilutions were made in 96-well polystyrene round bottom microwell plates with 50 µL final volume for each concentration. PBS+/+ was used as untreated control. Next, 50 µL bacteria solutions were added to each well to a final bacteria concentration of $5 \times 10^5$ CFU/mL. Plates were incubated at 37°C in Cytation 1 Cell Imaging Multimode Reader. Growth curves were obtained by measuring optical densities at 600 nm every 10 minutes for 18 hours using Cytation 1 Cell Imaging Multimode Reader. In addition, visual inspection of the plates was carried out after 18 hours and photographs were taken. MICs were defined as the lowest drug concentration in the microwell at which bacterial growth was not visible at the end of the incubation period. Besides MIC determination, specificity of TOUCAN 7mGe was also shown with subcultures on chromogenic media. For this purpose, 5 µL of different concentrations of each strain were inoculated onto CHROM agar Orientation Media (Becton Dickinson) and the bacterial growth of each bacteria strain tested was observed by naked eye after incubation at 37°C for 24 hours. Finally, photographs of the chromogenic media were taken.

**Calculation of equivalent concentration of NA and TOUCANs.** The stock solutions for Gemcitabine and Floxuridine were prepared to reach a concentration of 1mg/mL. The stock solution of TOUCANs were calculated as described below: For each TOUCAN we first calculated their concentration factor **(CF)**:

$$CF = MW \ TOUCAN \ / \ MW \ NA$$

7mGe CF = 2152 g/mol / 263,19 g/mol = 8,18
7mFx **CF** = 2134 g/mol / 246,19 g/mol = 8,67

Next, TOUCANS with equivalent amount of NA **(E)** were prepared as follows:

$$E = NA \ stock \ solution \ x \ CF$$

7mGe **E** = 1 mg/mL x 8,18 = **8,18 mg/mL,** containing 1mg/mL of Gemcitabine
7mFx **E** = 1 mg/m x 8,67 = **8,67 mg/mL,** containing 1mg/mL of Floxuridine

In this study, to facilitate the comparison and dose preparations of NAs and TOUCANs at equivalent concentrations, we have used 1mg/mL containing NA of each TOUCAN, along with 1mg/mL stock concentration of NAs.

**Calculation of TOUCAN Efficacy Ratio (TER).** TER was defined as the ratio between the MICs of each NAs and its respective TOUCAN.
**TER =** NA MIC / TOUCAN MIC
A lower TER indicates that the therapeutic efficacy of the TOUCAN is closer to the therapeutic efficacy of the naked nucleoside analogue, suggesting efficient activation by nucleases and efficient therapeutic outcome of a given TOUCAN.

***In vivo* assessment of the therapeutic efficacy of TOUCANs in a mouse infection model.**

All animal experiments described in this study were conducted at the Linköping University Center for Biomedical Resources and were approved by the Swedish Ethical Board "Jordbruksverket" under the animal protocol 1253. All experiments were conducted according to the principles expressed in the Declaration of Helsinki. Six-weeks old female b57/C7 mice were divided into three different groups: Placebo (saline solution), Floxuridine alone and TOUCAN 7mFx, with 8 mice per group. First, to induce the infection, *S. aureus* ATCC1766 strain was cultured on tryptone soy agar with 5% defibrinated sheep blood, and single colonies were transferred to 25 mL tryptone soy broth and incubated at 37°C for 12 hours with 200 rpm shaking. Next, bacteria solution was diluted 1/100 using PBS+/+ (~$10^7$ CFU/mL) and 50 μL (~$5x10^5$ CFU) were injected into the right thigh muscles of all mice under sterile conditions and 2% isoflurane anesthesia. This method was optimized based on our previous animal infection model.[16] In this model, the left uninfected leg was used as control for each animal. This model induces reproducible infections after 24 hours. Next, the therapeutic regime was adapted from a previous study that validated the efficacy of several antibiotics against *S. aureus*.[28] We have treated the mice for 24 hours with four doses of TOUCAN 7mFx equivalent to 5 mg/kg of Floxuridine in 100 μL of PBS-/-, administered intravenously by tail vein injections. The first dose was administered after 2 hours post-inoculation, followed by 3 additional injections at the same concentration every 6 hours. In total, each animal was given TOUCAN 7mFx equivalent to 20 mg/kg/24 hour of Floxuridine. In this study, two additional groups, Placebo and Floxuridine alone, were also tested in infected animals. In the Placebo group, mice were injected with a saline solution (PBS), and in the Floxuridine group, mice were injected with 20 mg/kg/24 hours of Floxuridine under the same conditions indicated for TOUCAN 7mFx. After 24 hours, blood samples were collected, and mice were euthanized. For a thorough blood analysis, we have included an additional group of uninfected/untreated mice as control. Next, gross pictures of both legs were taken and samples for histopathological and bacteriological analyses were collected. At least four mice per group per analysis were used in this study.

**Complete blood counts.** The mice were anesthetized by an intraperitoneal injection with a combination of 75 mg/Kg Ketamine and 1 mg/Kg of Dexmedetomidine. The efficacy of the anesthesia was confirmed by the loss of righting reflex of the animals. Next, mice were dorsally placed on the surgery pad and a 29-gauge needle fitted to a 1mL syringe was slowly inserted below the sternum. Once the needle reached the mouse hearth and blood appeared in the syringe, at least 400 μL of blood were extracted. Next, mice were euthanized. The needle was removed from the syringe and the blood was transferred to vials with EDTA to perform complete blood analysis using the IDEXX ProCyte Dx hematology analyzer.

**Gross pictures.** Once the mice were euthanized, the skin from legs and dorsal part was removed very carefully to allow observation of the bacterial infection and the associated tissue damage. Next, photographs were taken to document the localized infection for each animal.

**Histopathological analysis.** To evaluate the histopathological features of infected and uninfected tissues, the thigh muscles of the animals were dissected. Subsequently, tissues were transferred to 4% buffered formaldehyde solution and stored at 4°C for 24 hours. Then, tissues were removed from the formaldehyde solution and transferred into 50% ethanol for 15 minutes followed by 70% ethanol until processed. Next, tissues were fixed, dehydrated and wax infiltrated using a Leica TP1020 system for 7 hours. Subsequently, tissues were embedded in paraffin using a Leica EG 1150 system. The sections slides were prepared using an Automatic Rotary Microtome HM355S system and finally, samples were stained with Hematoxylin-Eosin (H&E) using an Automatic Leica ST4020 Linear Stainer. The slides were imaged using an Olympus BX51 light microscope.

**Bacteria counts.** To measure viable bacteria in mice, infected and uninfected tissues from both legs were collected, transferred to individual 1.5 mL vials and weighted on a microbalance. Each sample was homogenized in 1 mL of PBS using Fisherbrand Bead Mill 4 automatic homogenizer (Fisher Scientific). The homogenized solutions were serially diluted to 1:100, 1:1.000, 1:10.000 and 1:100.000. Next, 10 μL of each dilution were inoculated onto three different blood agar plates (TSA supplemented with 5% sheep blood) and incubated for 24 hours at 37°C. After incubation, the bacteria colonies were counted using a Colyte 3 HD automatic colony counter (Synbiosis) and were reported as Colony Forming Units per gram tissue (CFU/g).

**TOUCAN 7mFx on Gram-negative bacteria**

Floxuridine and equivalent amount of 7mFx solution were prepare as described before. As pretreatment, 7mFx was incubated with 10 U of micrococcal nuclease (MN) and incubated at 37oC for 1 hour (7mFx+MN). Next, 100 μL of PBS (untreated), Floxuridine, 7mFx or 7mFx+MN were serial diluted and loaded 96-well plate at the concentration indicated in the figures. Then, Bacteria cultures of of E. coli and P. mirabilis were adjusted to 106 CFU/mL for each strain and mixed in untreated of treated solutions. The plate was incubated at 37oC for 18 hours with every 10 minutes data acquisition using Cytation 1 Cell Imaging Multi-Mode Reader. At the end of the incubation period, minimal inhibitory concentrations of each drug for each strain were determined as the lowest drug concentration with no visible growth of bacteria.

**Anti-cancer drug susceptibility testing for TOUCAN 7mGe.** Gemcitabine and equivalent 7mGe solutions were prepared as explained before. Then, 7mGe was pre-exposed to 10 μl of 10 U MN for 1 hour at 37°C (7mGe+MN).

100 μl of both drugs (Gemcitabine and 7mGe+MN) were added to the related wells and serially diluted using PBS+/+ making the final concentrations between 100-2 μg/μl. Breast cancer cells (MDAMB231) and Pancreatic cancer cells (ASPC-1) were prepared as 10.000 cells/ml and 100 μl cell suspensions were added to each well. The plate was incubated at 37°C for 72 hours. At 48th hour of incubation, 100 μl CyQUANT® Direct Cell Proliferation Assay kit was added to each well. Cell counts and images were taken at the end of 72 hours incubation period using Cytation 1 Cell Imaging Multi-Mode Reader.

**Anti-cancer drug susceptibility testing for TOUCAN 7mDx.** Doxorubicin and equivalent 7mDx solutions were prepared as explained before. Then, 7mDx was pre-exposed to 5 μl of 10 U MN for 1 hour at 37°C (7mDx+MN). 100 μl of all drugs (Doxorubicin, 7mDx, and 7mDx+MN) were added to the related wells and serially diluted using PBS+/+ making the final concentrations between 20-0,3 μg/μl. Breast cancer cells (MDAMB231) were prepared as 20.000 cells/ml and 100 μl cell suspensions were added to each well. The plate was incubated at 37°C for 96 hours. At 48th hour of incubation, 100 μl CyQUANT® Direct Cell Proliferation Assay kit was added to each well. Cell counts and images were taken at the end of 96 hours incubation period using Cytation 1 Cell Imaging Multi-Mode Reader.

**Statistics.** Statistical analysis was performed in GraphPad Prism (GraphPad). All data were reported as mean ± SD.

**References**

1. Hutchings, M.I., Truman, A.W. & Wilkinson, B. Antibiotics: past, present and future. Curr Opin Microbiol 51, 72-80 (2019).
2. McKenna, M. Antibiotic resistance: the last resort. Nature 499, 394-396 (2013).
3. Theuretzbacher, U. Antibiotic innovation for future public health needs. Clin Microbiol Infect 23, 713-717 (2017).
4. Naran, K., Nundalall, T., Chetty, S. & Barth, S. Principles of Immunotherapy: Implications for Treatment Strategies in Cancer and Infectious Diseases. Front Microbiol 9, 3158 (2018).
5. Wang, J. et al. Antimicrobial Peptides Derived from Fusion Peptides of Influenza A Viruses, a Promising Approach to Designing Potent Antimicrobial Agents. Chem Biol Drug Des 86, 487-495 (2015).
6. Cal, P.M., Matos, M.J. & Bernardes, G.J. Trends in therapeutic drug conjugates for bacterial diseases: a patent review. Expert Opin Ther Pat 27, 179-189 (2017).
7. Lin, D.M., Koskella, B. & Lin, H.C. Phage therapy: An alternative to antibiotics in the age of multi-drug resistance. World J Gastrointest Pharmacol Ther 8, 162-173 (2017).
8. Gholizadeh, P. et al. How CRISPR-Cas System Could Be Used to Combat Antimicrobial Resistance. Infect Drug Resist 13, 1111-1121 (2020).
9. Theuretzbacher, U., Outterson, K., Engel, A. & Karlen, A. The global preclinical antibacterial pipeline. Nat Rev Microbiol 18, 275-285 (2020).
10. Rosenblum, D., Joshi, N., Tao, W., Karp, J.M. & Peer, D. Progress and challenges towards targeted delivery of cancer therapeutics. Nat Commun 9, 1410 (2018).
11. Feliu, J. et al. Management of the toxicity of chemotherapy and targeted therapies in elderly cancer patients. Clin Transl Oncol 22, 457-467 (2020).
12. Dugger, S.A., Platt, A. & Goldstein, D.B. Drug development in the era of precision medicine. Nat Rev Drug Discov 17, 183-196 (2018).
13. Mirza, A.Z. Advancement in the development of heterocyclic nucleosides for the treatment of cancer - A review. Nucleosides Nucleotides Nucleic Acids 38, 836-857 (2019).
14. Yates, M.K. & Seley-Radtke, K.L. The evolution of antiviral nucleoside analogues: A review for chemists and non-chemists. Part II: Complex modifications to the nucleoside scaffold. Antiviral Res 162, 5-21 (2019).
15. Tsume, Y., Borras Bermejo, B. & Amidon, G.L. The dipeptide monoester prodrugs of floxuridine and gemcitabine-feasibility of orally administrable nucleoside analogs. Pharmaceuticals (Basel) 7, 169-191 (2014).
16. Hernandez, F.J. et al. Noninvasive imaging of Staphylococcus aureus infections with a nuclease-activated probe. Nat Med 20, 301-306 (2014).
17. Thomson, J.M. & Lamont, I.L. Nucleoside Analogues as Antibacterial Agents. Front Microbiol 10, 952 (2019).
18. Principe, D.R. et al. Long-Term Gemcitabine Treatment Reshapes the Pancreatic Tumor Microenvironment and Sensitizes Murine Carcinoma to Combination Immunotherapy. Cancer Res 80, 3101-3115 (2020).
19. Hryciuk, B. et al. Severe acute toxicity following gemcitabine administration: A report of four cases with cytidine deaminase polymorphisms evaluation. Oncol Lett 15, 1912-1916 (2018).
20. Jordheim, L.P. et al. Gemcitabine is active against clinical multiresistant Staphylococcus aureus strains and is synergistic with gentamicin. Int J Antimicrob Agents 39, 444-447 (2012).
21. Sandrini, M.P., Shannon, O., Clausen, A.R., Bjorck, L. & Piskur, J. Deoxyribonucleoside kinases activate nucleoside antibiotics in severely pathogenic bacteria. Antimicrob Agents Chemother 51, 2726-2732 (2007).

22. Ciccolini, J., Serdjebi, C., Peters, G.J. & Giovannetti, E. Pharmacokinetics and pharmacogenetics of Gemcitabine as a mainstay in adult and pediatric oncology: an EORTC-PAMM perspective. Cancer Chemoth Pharm 78, 1-12 (2016).

23. Baker, J.A. et al. Pharmacogenomics of gemcitabine metabolism: functional analysis of genetic variants in cytidine deaminase and deoxycytidine kinase. Drug Metab Dispos 41, 541-545 (2013).

24. Mitra, A.K. et al. Pathway-based pharmacogenomics of gemcitabine pharmacokinetics in patients with solid tumors. Pharmacogenomics 13, 1009-1021 (2012).

25. Stokes, J.M., Lopatkin, A.J., Lobritz, M.A. & Collins, J.J. Bacterial Metabolism and Antibiotic Efficacy. Cell Metab 30, 251-259 (2019).

26. Koczor, C.A., Torres, R.A. & Lewis, W. The role of transporters in the toxicity of nucleoside and nucleotide analogs. Expert Opin Drug Metab Toxicol 8, 665-676 (2012).

27. Tsesmetzis, N., Paulin, C.B.J., Rudd, S.G. & Herold, N. Nucleobase and Nucleoside Analogues: Resistance and Re-Sensitisation at the Level of Pharmacokinetics, Pharmacodynamics and Metabolism. Cancers (Basel) 10 (2018).

28. Lee, D.G., Murakami, Y., Andes, D.R. & Craig, W.A. Inoculum Effects of Ceftobiprole, Daptomycin, Linezolid, and Vancomycin with Staphylococcus aureus and Streptococcus pneumoniae at Inocula of 10(5) and 10(7) CFU Injected into Opposite Thighs of Neutropenic Mice. Antimicrob Agents Ch 57, 1434-1441 (2013).

**Claims**

1. A drug delivery system which comprises a polynucleotide sequence susceptible of being cleaved by a specific nuclease derived from a bacterium or a mammalian cell, wherein the polynucleotide sequence **comprises** i) an oligonucleotide sequence of RNase-resistant modified RNA bases and/or nuclease-resistant DNA bases, acting as a resistant moiety, directly linked to ii) a polynucleotide sequence susceptible of being cleaved by a specific nuclease derived from a bacterium or a mammalian cell; wherein the polynucleotide sequence susceptible of being cleaved by a specific nuclease derived from a bacterium or a mammalian cell is in turn directly linked to a pharmacological active ingredient, wherein the polynucleotide sequence when cleaved at the cleaving site, releases the pharmacological active ingredient.

2. The drug delivery system of claim 1, wherein the polynucleotide sequence, from (upstream to downstream), **consists of** i) an oligonucleotide sequence of RNase-resistant modified RNA bases, or nuclease-resistant DNA bases, acting as a resistant moiety directly linked to ii) a polynucleotide sequence susceptible of being cleaved by a specific nuclease derived from a bacterium or a mammalian cell, wherein the polynucleotide sequence susceptible of being cleaved by a specific nuclease is directly linked to a pharmacological active ingredient, wherein the polynucleotide sequence when cleaved at the cleaving site, releases the pharmacological active ingredient.

3. The drug delivery system of claim 1 or 2, wherein the oligonucleotide sequence of RNase-resistant modified RNA bases, or nuclease-resistant DNA bases, acting as a resistant moiety, comprises or consists of chemically modified nucleotides (eg. 2'-O-Methyl, LNA, 2'-Fluoro)

4. The drug delivery system of any of claims 1 to 3, wherein the polynucleotide sequence susceptible of being cleaved by a specific nuclease derived from a bacterium or a mammalian cell is selected from the list consisting of mUmUmUmUTT-(pharmacological active ingredient) for S. aureus, mUmUmUmU-U-(pharmacological active ingredient) for coronavirus, and mUmUmUmUfAfAfA-(pharmacological active ingredient) for cancer cells such as pancreatic/breast cancer cells.

5. The drug delivery system of any of claims 1 to 4, wherein the pharmacological active ingredient is selected from the list consisting of alkylating agents, kinase inhibitor drugs, vinca alkaloids, anthracyclines, antimetabolites, aromatase inhibitors, topoisomerase inhibitors, mTOR inhibitors, retinoids, histone deacetylase inhibitors, and PARP inhibitors.

6. The drug delivery system of claim 5, wherein the pharmacological active ingredient is selected from the list consisting of a floxuridine, doxorubicin and gemcitabine.

7. The drug delivery system of any of claims 1 to 6, wherein the polynucleotide sequence is 2-30 nucleotides in length.

8. The drug delivery system of any of claims 1 to 6, wherein the polynucleotide sequence comprises i) an oligonucleotide sequence of RNase-resistant modified RNA bases and/or nuclease-resistant DNA bases, having 2-24 nucleotides in length, directly linked to ii) a polynucleotide sequence susceptible of being cleaved by a specific nuclease derived from a bacterium or a mammalian cell, having 1-5 nucleotides in length.

9. The drug delivery system of any of claims 1 to 6, wherein the polynucleotide sequence comprises i) an oligonucleotide sequence of RNase-resistant modified RNA bases and/or nuclease-resistant DNA bases, having 4 nucleotides in length, directly linked to ii) a polynucleotide sequence susceptible of being cleaved by a specific nuclease derived from a bacterium or a mammalian cell, having 2 nucleotides in length.

10. The drug delivery system of any of claims 7 to 9, wherein the polynucleotide sequence susceptible of being cleaved by a specific nuclease derived from a bacterium or a mammalian cell is linked to the pharmacological active ingredient by using phosphoramidite synthesis.

11. The drug delivery system of any of claims 1 to 6, wherein the polynucleotide sequence comprises i) an oligonucleotide sequence of RNase-resistant modified RNA bases and/or nuclease-resistant DNA bases, having 4 nucleotides in length, directly linked to ii) a polynucleotide sequence susceptible of being cleaved by a specific nuclease derived from a bacterium or a mammalian cell, having 2 nucleotides in length, wherein the polynucleotide sequence susceptible of being cleaved by a specific nuclease derived from a bacterium or a mammalian cell is linked to the pharmacological active ingredient by phosphoamidite synthesis, and wherein the oligonucleotide sequence of RNase-resistant modified RNA bases, or nuclease-resistant DNA bases, acting as a resistant moiety comprises or consists of 2'-O-Methyl nucleosides (-mC- -mU- or -mG-).

12. The drug delivery system of any of claims 1 to 11, wherein the pharmacological active ingredient is a bacteriostatic or bactericidal compound and the polynucleotide sequence is susceptible of being cleaved by a specific nuclease derived from a bacterium.

13. The drug delivery system of any of claims 1 to 11, wherein the pharmacological active ingredient is selected from the list consisting of a floxuridine, doxorubicin and gemcitabine and the polynucleotide sequence is susceptible of being cleaved by a specific nuclease derived from a bacterium.

14. The drug delivery system of any of claims 1 to 11, wherein the pharmacological active ingredient is selected from the list consisting of a floxuridine, doxorubicin and gemcitabine and the polynucleotide sequence is susceptible of being cleaved by a specific nuclease derived from a mammalian cell.

15. The drug delivery system of any of claims 1 to 11, wherein the drug delivery system is selected from the group consisting of mUmUmUmUTT directly linked to Gemcitabine or Floxuridine for S. aureus, mUmUmUmU-U directly linked to Resdisivir for coronavirus, and mUmUmUmUfAfAfA directly linked to Gemcitabine or Doxorubicin for pancreatic/breast cancer.

Figure 1. Scheme and proof of concept of TOUCAN technology

EP 4 000 635 A1

Figure 2.

Figure 3.

Figure 4.

Figure 5.

**Figure 6.**

a)

b)

**Figure 7**.

Figure 8.

Figure 9.

Figure 10.

**Figure 11.**

a)

b)

**Figure 12.**

Figure 13.

**Figure 14.**

**a.**

Legend:
- Untreated cells
- Gemcitabine (50 µg/mL)
- 7mGe+MN (= 50 µg/mL Ge)

**b.**

Untreated

**c.**

Gemcitabine

**d.**

7mGe +MN

Figure 15.

## EUROPEAN SEARCH REPORT

Application Number

**EP 20 20 7850**

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | GHIMIRE ANANTA ET AL: "Micrococcal-Nuclease-Triggered On-Demand Release of Vancomycin from Intramedullary Implant Coating Eradicates Staphylococcus aureus Infection in Mouse Femoral Canals", ACS CENTRAL SCIENCE, vol. 5, no. 12, 10 December 2019 (2019-12-10), pages 1929-1936, XP055792173, ISSN: 2374-7943, DOI: 10.1021/acscentsci.9b00870 Retrieved from the Internet: URL:http://pubs.acs.org/doi/pdf/10.1021/acscentsci.9b00870> * page 1930, column 2; figure 1 * | 1-15 | INV. A61K47/50 C07H21/02 C12Q1/6806 |
| A | HERNANDEZ FRANK J. ET AL: "NanoKeepers: stimuli responsive nanocapsules for programmed specific targeting and drug delivery", CHEMICAL COMMUNICATIONS, vol. 50, no. 67, 1 January 2014 (2014-01-01), pages 9489-9492, XP055792170, ISSN: 1359-7345, DOI: 10.1039/C4CC04248D Retrieved from the Internet: URL:https://www.researchgate.net/profile/Luiza-Hernandez-2/publication/263815672_NanoKeepers_Stimuli_responsive_nanocapsules_for_programmed_specific_targeting_and_drug_delivery/links/0c96053c02ab4506f9000000/NanoKeepers-Stimuli-responsive-nanocapsules-for-programmed-specific-targeting-and-drug-delivery.> * figure 1 * | 1-15 | TECHNICAL FIELDS SEARCHED (IPC) A61K C07H C12Q |

-/--

The present search report has been drawn up for all claims

| Place of search | Date of completion of the search | Examiner |
|---|---|---|
| The Hague | 6 May 2021 | Aslund, Fredrik |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

## EUROPEAN SEARCH REPORT

Application Number

EP 20 20 7850

Europäisches
Patentamt
European
Patent Office
Office européen
des brevets

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | PARK JUN YOUNG ET AL: "Targeted Therapy of Hepatocellular Carcinoma Using Gemcitabine-Incorporated GPC3 Aptamer", PHARMACEUTICS, vol. 12, no. 10, 18 October 2020 (2020-10-18), page 985, XP055792266, DOI: 10.3390/pharmaceutics12100985 Retrieved from the Internet: URL:https://www.ncbi.nlm.nih.gov/pmc/artic les/PMC7588995/pdf/pharmaceutics-12-00985. pdf> * page 5, last paragraph * ----- | 1-15 | |

TECHNICAL FIELDS
SEARCHED     (IPC)

The present search report has been drawn up for all claims

| Place of search | Date of completion of the search | Examiner |
|---|---|---|
| The Hague | 6 May 2021 | Aslund, Fredrik |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2

**EP 4 000 635 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6773885 B

- US 7803536 B


**Non-patent literature cited in the description**

- **HUTCHINGS, M.I. ; TRUMAN, A.W. ; WILKINSON, B.** Antibiotics: past, present and future. *Curr Opin Microbiol,* 2019, vol. 51, 72-80
- **MCKENNA, M.** Antibiotic resistance: the last resort. *Nature,* 2013, vol. 499, 394-396
- **THEURETZBACHER, U.** Antibiotic innovation for future public health needs. *Clin Microbiol Infect,* 2017, vol. 23, 713-717
- **NARAN, K. ; NUNDALALL, T. ; CHETTY, S. ; BARTH, S.** Principles of Immunotherapy: Implications for Treatment Strategies in Cancer and Infectious Diseases. *Front Microbiol,* 2018, vol. 9, 3158
- **WANG, J. et al.** Antimicrobial Peptides Derived from Fusion Peptides of Influenza A Viruses, a Promising Approach to Designing Potent Antimicrobial Agents. *Chem Biol Drug Des,* 2015, vol. 86, 487-495
- **CAL, P.M. ; MATOS, M.J. ; BERNARDES, G.J.** Trends in therapeutic drug conjugates for bacterial diseases: a patent review. *Expert Opin Ther Pat,* 2017, vol. 27, 179-189
- **LIN, D.M. ; KOSKELLA, B. ; LIN, H.C.** Phage therapy: An alternative to antibiotics in the age of multi-drug resistance. *World J Gastroint Pharmacol Ther,* 2017, vol. 8, 162-173
- **GHOLIZADEH, P. et al.** How CRISPR-Cas System Could Be Used to Combat Antimicrobial Resistance. *Infect Drug Resist,* 2020, vol. 13, 1111-1121
- **THEURETZBACHER, U. ; OUTTERSON, K. ; ENGEL, A. ; KARLEN, A.** The global preclinical antibacterial pipeline. *Nat Rev Microbiol,* 2020, vol. 18, 275-285
- **ROSENBLUM, D. ; JOSHI, N. ; TAO, W. ; KARP, J.M. ; PEER, D.** Progress and challenges towards targeted delivery of cancer therapeutics. *Nat Commun,* 2018, vol. 9, 1410
- **FELIU, J. et al.** Management of the toxicity of chemotherapy and targeted therapies in elderly cancer patients. *Clin Transl Oncol,* 2020, vol. 22, 457-467
- **DUGGER, S.A. ; PLATT, A ; GOLDSTEIN, D.B.** Drug development in the era of precision medicine. *Nat Rev Drug Discov,* 2018, vol. 17, 183-196

- **MIRZA, A.Z.** Advancement in the development of heterocyclic nucleosides for the treatment of cancer - A review. *Nucleosides Nucleotides Nucleic Acids,* 2019, vol. 38, 836-857
- **YATES, M.K. ; SELEY-RADTKE, K.L.** The evolution of antiviral nucleoside analogues: A review for chemists and non-chemists. Part II: Complex modifications to the nucleoside scaffold. *Antiviral Res,* 2019, vol. 162, 5-21
- **TSUME, Y. ; BORRAS BERMEJO, B. ; AMIDON, G.L.** The dipeptide monoester prodrugs of floxuridine and gemcitabine-feasibility of orally administrable nucleoside analogs. *Pharmaceuticals (Basel),* 2014, vol. 7, 169-191
- **HERNANDEZ, F.J. et al.** Noninvasive imaging of Staphylococcus aureus infections with a nuclease-activated probe. *Nat Med,* 2014, vol. 20, 301-306
- **THOMSON, J.M. ; LAMONT, I.L.** Nucleoside Analogues as Antibacterial Agents. *Front Microbiol,* 2019, vol. 10, 952
- **PRINCIPE, D.R. et al.** Long-Term Gemcitabine Treatment Reshapes the Pancreatic Tumor Microenvironment and Sensitizes Murine Carcinoma to Combination Immunotherapy. *Cancer Res,* 2020, vol. 80, 3101-3115
- **HRYCIUK, B. et al.** Severe acute toxicity following gemcitabine administration: A report of four cases with cytidine deaminase polymorphisms evaluation. *Oncol Lett,* 2018, vol. 15, 1912-1916
- **JORDHEIM, L.P. et al.** Gemcitabine is active against clinical multiresistant Staphylococcus aureus strains and is synergistic with gentamicin. *Int J Antimicrob Agents,* 2012, vol. 39, 444-447
- **SANDRINI, M.P. ; SHANNON, O. ; CLAUSEN, A.R. ; BJORCK, L. ; PISKUR, J.** Deoxyribonucleoside kinases activate nucleoside antibiotics in severely pathogenic bacteria. *Antimicrob Agents Chemother,* 2007, vol. 51, 2726-2732
- **CICCOLINI, J. ; SERDJEBI, C ; PETERS, G.J. ; GIOVANNETTI, E.** Pharmacokinetics and pharmacogenetics of Gemcitabine as a mainstay in adult and pediatric oncology: an EORTC-PAMM perspective. *Cancer Chemoth Pharm,* 2016, vol. 78, 1-12

- **BAKER, J.A. et al.** Pharmacogenomics of gemcitabine metabolism: functional analysis of genetic variants in cytidine deaminase and deoxycytidine kinase. *Drug Metab Dispos,* 2013, vol. 41, 541-545
- **MITRA, A.K. et al.** Pathway-based pharmacogenomics of gemcitabine pharmacokinetics in patients with solid tumors. *Pharmacogenomics,* 2012, vol. 13, 1009-1021
- **STOKES, J.M. ; LOPATKIN, A.J. ; LOBRITZ, M.A. ; COLLINS, J.J.** Bacterial Metabolism and Antibiotic Efficacy. *Cell Metab,* 2019, vol. 30, 251-259
- **KOCZOR, C.A. ; TORRES, R.A. ; LEWIS, W.** The role of transporters in the toxicity of nucleoside and nucleotide analogs. *Expert Opin Drug Metab Toxicol,* 2012, vol. 8, 665-676
- **TSESMETZIS, N. ; PAULIN, C.B.J. ; RUDD, S.G. ; HEROLD, N.** Nucleobase and Nucleoside Analogues: Resistance and Re-Sensitisation at the Level of Pharmacokinetics, Pharmacodynamics and Metabolism. *Cancers (Basel),* 2018, vol. 10
- **LEE, D.G. ; MURAKAMI, Y. ; ANDES, D.R. ; CRAIG, W.A.** Inoculum Effects of Ceftobiprole, Daptomycin, Linezolid, and Vancomycin with Staphylococcus aureus and Streptococcus pneumoniae at Inocula of 10(5) and 10(7) CFU Injected into Opposite Thighs of Neutropenic Mice. *Antimicrob Agents Ch,* 2013, vol. 57, 1434-1441